# EUROPÄISCHE PATENTSCHRIFT

(11) **EP 2 761 022 B1**
(45) Veröffentlichungstag und Bekanntmachung des Hinweises auf die Patenterteilung: **23.11.2022**
(21) Anmeldenummer: 12770456.7
(22) Anmeldetag: 01.10.2012
(51) Int. Cl.: C12Q 1/6837

(54) **VERFAHREN ZUR RÄUMLICHEN ANORDNUNG VON PROBENFRAGMENTEN ZUR AMPLIFIKATION UND IMMOBILISIERUNG FÜR WEITERE DERIVATISIERUNGEN**
METHOD FOR THE SPATIAL ARRANGEMENT OF SAMPLE FRAGMENTS FOR AMPLIFICATION AND IMMOBILIZATION FOR FURTHER DERIVATIZATIONS
PROCÉDÉ D'AGENCEMENT SPATIAL DE FRAGMENTS D'ÉCHANTILLONS POUR LES AMPLIFIER ET LES IMMOBILISER EN VUE DE DÉRIVATISATIONS ADDITIONNELLES

(30) Priorität: 30.09.2011 DE 102011054101
(43) Veröffentlichungstag der Anmeldung: 06.08.2014
(73) Patentinhaber: BioCopy GmbH, 79312 Emmendingen (DE)
(72) Erfinder: HOFFMANN, Jochen, 71229 Leonberg (DE); ROTH, Günter, 79110 Freiburg (DE)
(74) Vertreter: Hertin und Partner Rechts- und Patentanwälte PartG mbB
(86) Internationale Anmeldenummer: PCT/EP2012/069359
(87) Internationale Veröffentlichungsnummer: WO 2013/045700

(56) Entgegenhaltungen:
- WO-A1-2010/100265
- WO-A2-01/27327
- WO-A2-2008/022332
- JOCHEN HOFFMANN ET AL: "Solid-phase PCR in a picowell array for immobilizing and arraying 100?000 PCR products to a microscope slide", LAB ON A CHIP, Bd. 12, Nr. 17, 1. Januar 2012 (2012-01-01), Seite 3049, XP055046774, ISSN: 1473-0197, DOI: 10.1039/c2lc40534b
- Hoffmann et al.: "SINGLE-MOLECULE PCR IN A PICOWELL ARRAY SIMULTANIOUSLY IMMOBILIZING PCR PRODUCTS TO A PDMS COVERSLIDE", 15th International Conference on Miniaturized Systems for Chemistry and Life Sciences October 2-6 2011 Seattle , USA , 2. Oktober 2011 (2011-10-02), Seiten 900-902, XP055047304, Gefunden im Internet: URL:http://www.rsc.org/images/LOC/2011/PDF s/Papers/302_0209.pdf [gefunden am 2012-12-10]

## Beschreibung

Die Erfindung betrifft ein Verfahren zur Durchführung einer biochemischen oder chemischen Reaktion für eine isolierte, räumlich getrennte Amplifikation von Probenfragmenten bei gleichzeitiger Immobilisierung und räumlichen Anordnung der Probenfragmente und Reaktionsprodukte, den Amplifikaten, an einer oder mehreren geeigneten Festphasen für anschließende Derivatisierungen.

Unter einer Amplifikationsreaktion versteht man die Herstellung von klonalen Kopien (Amplifikaten) eines definierten Ausgangsmaterials, bei dem es sich beispielsweise um DNA oder RNA unterschiedlicher biologischer Quellen in aufgereinigter und oft fragmentierter Form handelt. Die Amplifikate können demnach als Reaktionsprodukte der Amplifikationsreaktion bezeichnet werden. Im Stand der Technik werden zur Durchführung der Amplifikation oftmals Mikrokompartimente erzeugt, die aus einer wässrigen Lösung bestehen und von Öl umschlossen sind. Diese sogenannte Öl-in-Wasser Emulsion erlaubt eine räumliche und chemisch reaktive Trennung der Mikrokompartimente. Das Mikrokompartiment enthält ein Mikrokügelchen beschichtet mit Fängermolekülen, einen Amplifikationsmix und genau ein Probenfragment. Im Laufe der Amplifikationsreaktion werden bis zu 10⁷ identische DNAKopien des Probenfragments erzeugt und an die Oberfläche des Mikrokügelchens gebunden. Wenn der Amplifikationsmix ein PCR-Mix (PCR = polymerase chain reaction) ist wird dieser Prozess als Öl-Emulsions-PCR (emPCR) bezeichnet [Dressman et al., Proc. Natl. Acad. Sci. U. S. A., vol. 100, pp. 8817-8822, 2003].

Das Grundkonzept der Durchführung enzymatischer Amplifikationsreaktionen in Emulsionen wurde erstmals 1998 von Griffiths und Tawfik beschrieben [Tawfik und Griffiths, Nat Biotechnol, vol. 16, pp. 652-656, 1998]. Dieses Konzept wurde 2003 und 2004 angewendet, um eine Amplifikation von DNA-Probenfragmenten mittels PCR in Öl-Emulsionen [Nakano et al., Journal of Biotechnology, vol. 102, pp. 117-124, 2003] zu ermöglichen oder zur gleichzeitigen Immobilisierung der DNA-Amplifikate auf einem Mikrokügelchen [Dressman et al., Proc. Natl. Acad. Sci. U. S. A., vol. 100, pp. 8817-8822, 2003; Nakano et al., Journal of Bioscience and Bioengineering, vol. 99, pp. 293-295, 2005; Musyanovych, Mailander und Landfester, Biomacromolecules, vol. 6, pp. 1824-1828, 2005; Diehl et al., Nature Methods, vol. 3, pp. 551-559, 2006]. Mikrokügelchen bezeichnen hierbei monodisperse (identische Größe) Polymerkügelchen in üblichen Größenordnungen von 10 nm bis 100 µm. Die Oberfläche der Mikrokügelchen kann so aktiviert sein, dass vorzugsweise Fängermoleküle aber auch Amplifikate immobilisiert werden können. Hierbei sollen sowohl gebundene Fängermoleküle als auch Amplifikate in ihrer Funktionalität möglichst uneingeschränkt bleiben.

Die kleinste Kompartimentierung in der eine PCR demonstriert wurde, war in Liposomen (V = 3.3 aL) [Oberholzer, Albrizio und Luisi, Chemistry & Biology, vol. 2, pp. 677-682, 1995] und Wasser-in-Öl-Emulsionen (V = 1 aL) [Musyanovych, Mailander und Landfester, Biomacromolecules, vol. 6, pp. 1824-1828, 2005; Diehl et al., Nature Methods, vol. 3, pp. 551-559, 2006; Zeng et al., Anal. Chem., vol. 82, pp. 3183-3190, 2010; Novak et al., Angewandte Chemie-International Edition, vol. 50, pp. 390-395, 2011]. Nach dieser Methode ist es aber nicht möglich, eine definierte Anordnung der Reaktionskompartimente zu erzielen. Dies kann frühestens im Anschluss an die Reaktion durch Anordnung der Mikrokügelchen geschehen.

Aus dem Stand der Technik ist bekannt, dass die Gesamtheit aller Mikrokügelchen in einem separaten Schritt in einer regelmäßigen Anordnung auf eine Oberfläche/Trägermaterial gebracht werden können. Dies kann beispielsweise durch die Einbringung der Mikrokügelchen in einen Multiwell-Arrays erfolgen, beispielsweise Systeme GS FLX, GS FLX Titanium, GS Junior und PGM - Personal Genome Machine, oder durch Immobilisierung der Mikrokügelchen auf einer planaren Oberfläche mittels ebenfalls kommerziell erhältlicher Systeme, beispielsweise SOLiD und G.007 Polonator. Durch eine sich anschließende Derivatisierung, beispielsweise Sequenzierreaktion, lässt sich jeder Position eines Mikrokügelchens eine bestimmte Nukleinsäuresequenz zuordnen.

Die räumliche Trennung von Mikrokompartimenten für Amplifikationsreaktionen wird gegenwärtig hauptsächlich durch zwei Systeme realisiert. Neben der Kompartimentierung in Mikrotröpfchen wie z.B. der emPCR, die einzelne wässrige Reaktionsphasen durch eine Öl-Phase trennt, ist auch die Trennung in Mikrokavitäten im Stand der Technik beschrieben, wo beispielsweise einzelnen Amplifikationsbereiche durch eine Festphase voneinander getrennt werden.

In den letzten zwei Jahrzehnten wurden Systeme beschrieben, die eine PCR innerhalb einer mikrostrukturierten Festphase, einem mikrofluidischen Chip (on-Chip Mikroreaktoren), in entsprechenden Mikrokompartimenten in Reaktionsvolumina bis zu 1.3 pL untersucht haben [Leamon et al., Electrophoresis, vol. 24, pp. 3769-3777, 2003; Dube, Qin und Ramakrishnan, PLoS ONE, vol. 3, 2008; Marcus, Anderson und Quake, Anal. Chem., vol. 78, pp. 956-958, 2006; Matsubara et al. Bioelectron., vol. 20, pp. 1482-1490, 2005; Shen et al. Lab Chip, vol. 10, pp. 2666-2672, 2010; Nagai et al., Anal. Chem. 73, 2001, pp. 1043-1047]. Die Kompartimente erlauben dabei zwar eine Kompartimentierung und eine Amplifizierung der DNA, jedoch werden diese Kompartimente nicht einzeln befüllt. Es ist daher nicht möglich unterschiedliche Proben gleichzeitig zu testen und die entstehenden Amplifikate den entsprechenden Proben zuzuordnen. In der Offenbarung von Mitterer et al. ("Microarray-Based Identification of Bacteria in Clinical Samples by Solid-Phase PCR Amplification of 23S Ribosomal DNA Sequences", Journal of Microbiology, Mar. 2004, p 1048 - 1057) wird eine solche on-chip PCR beschrieben, wobei auch solid-phase Primer zum Einsatz kommen können. Auch hier ist es jedoch nicht möglich, unterschiedliche Proben auf den einzelnen Spots aufzutragen. Dieses Array wird über eine Öffnung befüllt, so dass sich die Probenfragmente über die einzelnen Spots verteilt. Eine Zuordnung zwischen Amplifikat und Original kann daher nicht erfolgen. Es ist lediglich möglich nachzuweisen, welche Amplifikate hergestellt wurden.

Die praktische Handhabung und Analytik von einzelnen Molekülen wird im Rahmen des Nachweises von einzelnen DNA-Fragmenten oft auch als Digitale-PCR bezeichnet [McCaughan und Dear, J. Pathol., vol. 220, pp. 297-306, 2010; Zhang und Da, Chem. Rev., vol. 110, pp. 4910-4947, 2010; Fair, Microfluid. Nanofluid., vol. 3, pp. 245-281, 2007]. Die Digitale PCR ist als Spezialfall der PCR in on-Chip Mikroreaktoren zu verstehen. Hierbei wird pro Mikrokompartiment genau ein oder nur wenige Probenfragmente, insbesondere genau ein oder nur wenige unterschiedliche DNA-Moleküle amplifiziert. Sofern ein DNA-Molekül vorhanden ist entsteht ein spezifisches Amplifikationsprodukt. Dieses Verfahren erlaubt das Erfassen einzelner identischer DNA-Moleküle in Form eines digitalen Zählens der positiven Amplifikationsreaktionen bzw. der entsprechend positiven Mikrokompartimente. Da die Amplifikationsreaktion auf einer PCR basiert, wird dies als Digitale PCR (dPCR) bezeichnet. Die erste dPCR in einem mikrostrukturierten Chip als Array von Mikrokompartimenten wurde 2004 von Matsubara et al. beschrieben [Matsubara et al., Anal. Chem., vol. 76, pp. 6434-6439, 2004]. Weitere Publikationen beschreiben dPCR in Mikroeaktoren [Shen et al., Lab Chip, vol. 10, pp. 2666-2672, 2010; Sundberg et al., Anal. Chem., vol. 82, pp. 1546-1550, 2010]. Mittels der dPCR kann die Anzahl von DNA-Molekülen in einer Probenlösung sehr genau bestimmt werden [Hirono-Hara, Noji und Takeuchi, J. Appl. Phys., vol. 105, pp. 102016-5, 2009; Bhat et al., Analytical und Bioanalytical Chemistry, vol. 394, pp. 457-467, 2009]. Dies kann auch zur Bestimmung der DNA-Konzentration bei der Sequenzierung eingesetzt werden [Dube, Qin und Ramakrishnan, PLoS ONE, vol. 3, 2008; White et al., Bmc Genomics, vol. 10, pp. 116, 2009]. Weiterhin kann die dPCR zur Erfassung von hochparalleler Genexpression eingesetzt werden [Fan und Quake, Anal. Chem., vol. 79, pp. 7576-7579, 2007; Warren et al., Proc. Natl. Acad. Sci. U. S. A., vol. 103, pp. 17807-17812, 2006]. Wie auch bei der PCR in on-Chip Mikroreaktoren erfolgt auch bei der digitalen PCR die Amplifikation einzelner Probenfragmente, eine Immobilisierung der Amplifikate findet jedoch nicht statt. Außerdem ist nachteilig, dass die dPCR mit einem hohen Kosten- und Zeitaufwand verbunden ist.

Die WO 2010/100265 A1 gehört zum Stand der Technik und beschreibt ein Verfahren zur Vervielfältigung von Probenfragmenten, welche in einem eine Vielzahl von Kompartimenten enthaltenden Mikroarray durchgeführt wird. In den Kompartimenten sind immobilisierte Fängermoleküle an denen ein Amplifikat gebunden wird.

Die WO 2008/022332 A2 gehört zum Stand der Technik und offenbart ebenfalls ein Verfahren zur Vervielfältigung von Probenfragmenten via Primerverlängerung, durchgeführt auf einem Mikroarray.

Die WO 01/27327 A2 gehört zum Stand der Technik und beschreibt einen Array aus Kavitäten in denen eine PCR durchgeführt wird und anschließend die Produkte an Fängermoleküle gebunden werden.Die beschriebenen Möglichkeiten der Amplifikation von Probenfragmenten, beispielsweise Nukleinsäuresequenzen, stellt eine vorgelagerte Reaktion dar, wenn heutige Sequenzierverfahren (Next-Generation Sequenziersysteme) zum Nachweis von beispielsweise Nukleinsäuresequenzen herangezogen werden. Bei derartigen Sequenzierverfahren werden die in der Amplifikationsreaktion erzeugten Amplifikate derivatisiert.

In US 6,300,070 ist ein Verfahren für die Amplifikation von einzelnen DNA-Molekülen auf Oberflächen beschrieben. Hier werden die DNA-Moleküle mit einem Ende kovalent an oberflächengebundenen DNA-Fängermolekülen angebunden. Nachfolgend wird das zweite Ende der DNA-Moleküle mit einem benachbarten DNA-Fängermolekül verbunden. Bei diesen Reaktionen werden somit einzelne Probenfragmente lokal amplifiziert und räumlich immobilisiert (auch als Brückenamplifikation oder Bridge-Amplifikation bezeichnet). Es gibt jedoch keine physikalische Unterteilung in Kompartimente, sodass es dazu kommen kann, dass die Amplifikationsbereiche ineinander laufen und so zwei oder mehrere Bereiche überlappen. Die Amplifikate können daher nicht mit ausreichender Sicherheit einem bestimmten Probenfragment zugeordnet werden. Außerdem ist nachteilig, dass nur der entstehende Strang und Gegenstrang immobilisiert werden können. Das Anbinden von nur einem Strang ist nicht möglich.

In der WO2009/0127589 ist eine Methode und Vorrichtung zur Sequenzierung von DNA basierend auf einem FET Array zur kalorimetrischen Bestimmung eines unbekannten DNA-Probenfragments beschrieben. Hierbei sind die Proben jedoch während der eigentlichen Reaktion nicht voneinander getrennt. Außerdem erfolgt die Immobilisierung außerhalb des Chips in Form von Öl-Emulsionen. Das beschriebene System ist dadurch sehr aufwendig und erlaubt keine ein-eindeutige Zuordnung von Proben und Amplifikaten.

Die US 6,001,568 offenbart ein Verfahren zur Amplifikation von einzelnen DNA-Molekülen bei gleichzeitiger Immobilisierung in einer gelartigen oder mit Mikroporen durchzogenen Festkörpermatrix. Probenfragmente lagern sich statistisch verteilt räumlich voneinander getrennt an, werden dort amplifiziert und immobilisiert. Nachteilig ist jedoch, dass die räumliche Trennung während der Reaktion nicht bestehen bleibt. Denn die Amplifikationsbereiche dehnen sich aus und wachsen ineinander, sodass eine spätere eindeutige Zuordnung der Amplifikate ausgeschlossen ist.

Weiterhin beschreibt die US 6,482,593 eine Methode und Vorrichtung für die Detektion von DNA-Molekülen mit Hilfe eines Sensorarrays. Dieses Array ist ein strukturiertes Multiwell- Array, das an diskreten Stellen immobilisierte Fängermoleküle (Biosensoren) aufweist. Eine Amplifikationsreaktion findet jedoch nicht statt.

Die US 7,244,559 offenbart ein Verfahren zur DNA Sequenzierung. Die zu sequenzierenden Probenfragmente werden mehrfach linear hintereinander gereiht, um ein verstärktes Signal von einer nachfolgenden Sequenzierreaktion zu erhalten. Allerdings erfolgt die Amplifikation in einem vorgelagerten Schritt außerhalb des Arrays. Dadurch wird das Verfahren aufwendig und fehleranfällig.

In der US 7,323,305 ist eine Methode zur Sequenzierung von DNA beschrieben. Die Probenfragmente werden mittels einer emPCR amplifiziert, wobei die Amplifikate auf Mikrokügelchen amplifiziert und immobilisiert werden. Es ist jedoch nicht möglich mehrere einzelne Proben parallel und getrennt voneinander zu amplifizieren. Außerdem ist nachteilig, dass kein Array erzeugt wird, mit dem nachfolgende Analysen vorgenommen werden können.

Nachteilig an diesem im Stand der Technik beschriebenen Verfahren ist vor allem, dass keine räumlich definierte Anordnung der Mikrokompartimente zueinander erfolgt. Hierfür ist ein separater Schritt notwendig, indem die Mikrokügelchen in eine definierte Anordnung zueinander verbracht werden. Oder aber es ist eine räumlich definierte Anordnung der Mikrokompartimente möglich, aber es findet keine Immobilisierung der Amplifikate statt. Die kommerziellen Systeme erlauben keine Modifikation, so dass beispielsweise ein Masterarray herstellbar wäre.

Die US 7,335,762 beschreibt ein Verfahren für die Sequenzierung von DNA, mit dem Fokus auf dem Auslesen der Sequenzierreaktion, wobei das Probenfragment an eine mobile Phase gebunden ist. Dieser Stand der Technik ist nicht zufriedenstellend, da keine Kompartimente vorgesehen sind, in welche unterschiedliche Proben aufgetragen werden können. Auch muss die Amplifikation vorgeschaltet werden, was den Zeit- und Kostenaufwand erhöht.

In der US 7,638,276 ist ein Verfahren für eine emPCR für die Herstellung von DNA-Klonen bei gleichzeitiger Immobilisierung auf Mikrokügelchen für die DNA Sequenzierung beschrieben. Auch hier können nicht mehrere Proben nebeneinander aufgetragen werden.

Aus der WO 2004/044240 ist ein Verfahren zum parallelen Nachweis unterschiedlicher Nukleinsäuren bekannt. Dabei werden Primer beim Vorhandensein der Nukleinsäure verlängert, wobei die Primer einen Zwischenabschnitt aufweisen. Dieser Zwischenabschnitt wird mittels eines weiteren Primerpaares amplifiziert und durch Hybridisierung mit einer Sonde nachgewiesen. Allerdings ist die Anzahl von DNA-Fragmenten die gleichzeitig amplifiziert und analysiert werden können beschränkt. Vor allem ist es nicht möglich eine ein-eindeutige Zuordnung zwischen der Probe-Nukleinsäure und dem entstehenden Amplifikat herzustellen. Es kann lediglich nachgewiesen werden, dass ein bestimmtes Amplifikat entstanden ist.

Aus der WO 2009/156915 ist ein Amplifikationsverfahren bekannt, welches eine Detektion der Amplifikate ermöglicht. Dabei werden die Amplifikate an Fängermoleküle gebunden, jedoch handelt es sich hierbei um keine kovalente Bindung, so dass die Bindung nur bei bestimmten Temperaturen zustande kommt und nicht dauerhaft besteht. Somit kann lediglich detektiert werden, dass ein bestimmtes Produkt entstanden ist. Nach der Detektion befindet sich das Amplifikat jedoch wieder in Lösung und steht nicht für nachfolgende Analysen zur Verfügung. Es kann daher kein Array hergestellt werden. Außerdem ist eine ein-eindeutige Zuordnung zwischen Produkt und Ausgangsmolekül bei der Verwendung mehrerer Ausgangsmoleküle nicht möglich.

Aus der Offenbarung von Mikhailovich et al. ("Identification of Rifampin-Resistant Mycobacterium tuberculosis Strains by Hybridization, PCR, and Ligase Detection Reaction on Oligonucleotide Microchips" J. of Clinic. Microbiology; 2001; p. 2531 - 2540) ist bekannt, dass Fängermoleküle in Gel immobilisiert sein können. Auch hier ist jedoch eine ein-eindeutige Zuordnung der Ampilfikate und der entsprechenden Proben nicht möglich, da die Amplifikate zunächst frei in Lösung sind und zu räumlich entfernteren Fängern diffundieren können.

Die im Stand der Technik beschriebene Next-Generation Sequenziersysteme weisen zahlreiche Nachteile auf. Diese Systeme benötigen vor dem eigentlichen Sequenzierprozess erstens eine Amplifizierung der Probenfragmente und zweitens eine räumliche Fixierung der Amplifikate, um ein diskretes Signal der Sequenzierreaktion zu erhalten. Das Kompartimentieren und Amplifizieren einzelner Probenfragmente wird durch die emPCR in einem ersten separaten Schritt erreicht. Die regelmäßige Anordnung der Amplifikate auf einer Festphase wird in einem zweiten separaten Schritt durch räumliche Fixierung der Mikrokügelchen auf einer Oberfläche erreicht. Das Verfahren der emPCR ist sehr aufwendig und geht mit einem Verlust an Probenfragmenten einher. Dies schränkt vor allem die Analyse von Mischungen aus Probenfragmenten, die nur wenige identische Kopien jedes Probenfragmentes enthalten (geringe Redundanz der Probe) deutlich ein. Dies gilt insbesondere für Einzel-Zell-Analysen. Bei kommerziellen Systemen wie beispielsweise GS FLX, GS FLX Titanium, GS Junior, SOLiD, PGM - Personal Genome Machine und G.007 Polonator werden die Amplifikate der Probenfragmente über eine emPCR hergestellt und dabei auf der Oberfläche von Beads (Mikrokügelchen) immobilisiert [A. Griffiths und D. Tawfik, US 7,638,276]. Anschließend werden die aufgereinigten Beads nach unterschiedlichen Mechanismen in einer regelmäßig Anordnung auf einer Oberfläche für die Sequenzierung fixiert. Die Durchführung einer emPCR ist mit Nachteilen behaftet, die zum einen durch die Kosten für DNA-Beads (Capture-Beads für die Immobilisierung von Probenfragmenten), magnetische Beads (Enrichment-Beads benötigt zur Aufreinigung nach der emPCR) und Chemikalien (Detergenzien, Puffer, Emulsionsöl, emPCR Reagenzien) begründet sind. Zum anderen werden Spezial-Laborgeräte für die Handhabung der Beads benötigt. Dazu zählen Partikel Counter/Zählsysteme, um die aufgereinigten Beads nach der emPCR zu quantifizieren. Auch die Durchführungszeit für einen emPCR-Prozess ist mit 11 bis 13 Stunden ein Nachteil bekannter Systeme. Ein expliziter Nachteil der emPCR besteht darin, dass die Amplifizierungs- und Immobilisierungseffizienz von der Länge der Probenfragmente abhängt. Je länger das Probenfragment, desto länger müssen die Haltezeiten der einzelnen Temperaturschritte der PCR eingestellt werden. Das hat zur Folge, dass die Wahrscheinlichkeit für die Fusion einzelner Öltröpfchen, insbesondere bei den hohen Temperaturen um 95°C, deutlich erhöht wird. Ein Verschmelzen der Tröpfchen löst aber die Kompartimentierung auf, was wiederum zu Verlust an "reinen" Probenfragmenten führt, da dann mind. 2 Probenfragmente vereint wurden. Als Resultat steht nach dem Prozess nur eine geringe Anzahl an reinen Amplifikat-Mikrokügelchen zur Verfügung. Daher ist man aktuell bei DNA auf eine Länge von ca. 500 Basenpaaren beschränkt.

In Sequenziersystemen, welche sich einer Bridge-Amplifikation bedienen, besteht kein Bedarf für eine emPCR, da die Amplifikation direkt auf einer mit Fängermolekülen beschichteten Oberfläche stattfindet. Nachteilig an diesem System ist die Tatsache, dass die einzelnen Amplifikat-Bereiche wachsen und so ineinander wachsen können. Somit ist keine vollständige Kompartimentierung gewährleistet. Weiterhin ist es von Nachteil, dass bei der Bridge-Amplifikation sowohl der sense als auch der Antisense-DNA-Strang auf der Oberfläche immobilisiert wird. Man erhält so eine Mischung beider DNA-Stränge.

In Systemen für eine digitale PCR wird eine räumliche Kompartimentierung sowohl des Probenfragments als auch nach der Amplifikation des Amplifikats durch ein mikrofluidisches System erreicht. Hierbei werden generell nur ein einziges oder wenige Probenfragmente amplifiziert. Eine Immobilisierung des Amplifikats und deren gleichzeitige räumlich definierte Anordnung sind bei der digitalen PCR nicht beschrieben.

Weiterhin treten bei den im Stand der Technik offenbarten Verfahren und Systemen häufig Kontaminationen auf, da die Kompartimente nur unzureichend gegeneinander abgeschlossen sind. Außerdem können die offenbarten Systeme nicht mittels Massenherstellungsverfahren bereitgestellt werden und sind zudem nicht mit anderen Biomolekülen kompatibel. Die Verwendung der Systeme beschränkt sich ausschließlich auf DNA.

Aufgabe der vorliegenden Erfindung ist es daher, ein neues Amplifikationsverfahren zu schaffen, was nicht die Nachteile und Mängel des Standes der Technik aufweist.

### Beschreibung

Gelöst wird die Aufgabe durch die unabhängigen Ansprüche. Vorteilhafte Ausführungsformen ergeben sich aus den Unteransprüchen.

Die Erfindung betrifft ein Verfahren zur Amplifikation von Nukleinsäuren, umfassend folgende Verfahrensschritte:
a. Bereitstellung einer Festphase mit Mikrostruktur, wobei die Mikrostruktur die Festphase so unterteilt, dass mehrere Kompartimente entstehen und wobei Fängermoleküle mit der Festphase verbunden sind, und wobei es sich bei der Festphase um eine Pikotiterplatte handelt
b. Einbringung mindestens eines Probenfragmentes in mindestens ein Kompartiment,
   wobei das mindestens eine Probenfragment an beiden Enden Adaptoren aufweist, wobei die Adaptoren eine einheitliche Sequenz mit einer Länge von bis zu 1000 Nukleotiden und/oder unterschiedliche funktionelle Einheiten aufweisen
c. Einbringung eines Reaktionsmixes für die Amplifikation in mindestens ein Kompartiment,
d. Abdeckung der einzelnen Kompartimente mit einer Abdeckung auf der Fängermoleküle immobilisiert sind,
e. Amplifikationsschritt, wobei jedes Probenfragment in dem entsprechenden abgeschlossenen Kompartiment eigenständig amplifiziert wird,
f. wobei die durch den Amplifikationsschritt generierten Amplifikate an den Fängermolekülen immobilisiert werden,
   wobei die Amplifikate nach der Immobilisierung an der Festphase sequenziert werden.

Das erfindungsgemäße Verfahren ist vorteilhaft gegenüber dem Stand der Technik, da eine Amplifikation der einzelnen Probenfragmente bei gleichzeitiger Immobilisierung der Amplifikate stattfindet. Dies geschieht durch die regelmäßige Anordnung der Fängermoleküle auf einer Festphase, wodurch beispielsweise eine emPCR umgangen werden kann und direkt die räumlich getrennte Amplifikation und Anordnung von Probenfragmenten erreicht wird. Dabei wird eine fragmentierte Probe in die mikrostrukturierte Festphase eingebracht. Eine Mikrostrukturierung und/oder Mikrostruktur bezeichnet im Sinne der Erfindung insbesondere eine Strukturierung der Oberfläche der Festphase im Nanometer- oder Mikrometerbereich. Eine Festphase bezeichnet im Sinne der Erfindung insbesondere einen festen Träger, in dem oder auf dem die Amplifikationsreaktion abläuft.

Die Festphase weist mehrere Kompartimente auf. Es ist bevorzugt, dass die Kompartimente als Mikrokompartimente ausgestaltet sind, die im Sinne der Erfindung Kompartimente im Mikrometerbereich sind. Bevorzugt weist die Festphase eine Mehrzahl an Mikrokompartimenten auf, wobei es bevorzugt ist, dass die Mehrzahl der Kompartimente jeweils genau eine Art von Probenfragment enthält. Das heißt, es ist bevorzugt, dass insbesondere in einem Kompartiment Fragmente einer Probe enthalten sind, so dass die Festphase, insbesondere die Kompartimente Fragmente zahlreicher gleicher oder unterschiedlicher Proben aufweisen kann.

An die mikrostrukturierte Oberfläche der Festphase sind Fängermoleküle gebunden. Es kann auch bevorzugt sein, dass die Oberfläche weitere Moleküle, insbesondere die Amplifikate bindet. Besonders bevorzugt ist, dass die Fängermoleküle an die Oberflächen in den Kompartimenten gebunden sind. Diese Ausführungsform liefert besonders gute Ergebnisse, da so sichergestellt wird, dass möglichst viele Fängermoleküle an Amplifikate binden können und so eine hohe Dichte an gebundenen Amplifikaten entsteht.

Das Verfahren der Erfindung zeichnet sich besonders dadurch aus, dass jedes Kompartiment separat befüllt werden kann und es nicht nur eine Befüllungsöffnung gibt, sodass sich das Probenfragment auf die unterschiedlichen Kompartimente verteilt. Dies bietet eine Vielzahl von Möglichkeiten, da nun eine hohe Anzahl an Proben parallel amplifiziert und untersucht werden kann.

Es kann außerdem bevorzugt sein, dass der Reaktionsmix, bevorzugt ein Amplifikationsmix, vor dem Probenfragment eingebracht wird. Der Fachmann weiß, welche Reihenfolge sich am besten für die jeweiligen Reagenzien und Anwendungen eignet, ohne selbst erfinderisch tätig zu werden.

Außerdem zeichnet sich dir Erfindung dadurch aus, dass jedes Probenfragment separat in seinem Kompartiment amplifiziert wird und es auch während der Reaktion zu keiner Vermischung oder Überschneidung der Amplifikationsbereiche kommen kann. Die strenge räumliche Trennung der Proben bleibt somit auch während der Reaktion erhalten und führt zu räumlich voneinander getrennten Amplifikaten. Die so bereitgestellten Amplifikate können im Sinne der Erfindung auch als Produkte des Amplifikationsschrittes oder der Amplifikationsreaktion bezeichnet werden.

In der vorliegenden Erfindung bezeichnet ein Amplifikat insbesondere die Gesamtheit aller durch eine Amplifikationsreaktion hergestellten Abbilder eines einzelnen Probenfragments. Es ist vorzugsweise identisch oder ein eindeutig ableitbares Derivat des Probenfragments. Das Amplifikat oder Teilabschnitte desselben können dabei von einem oder mehreren Teilabschnitten oder der Gesamtheit eines oder mehrerer Probenfragmente abgeleitet sein. Wenn für ein bevorzugtes Verfahren beispielsweise genomische DNA für die Amplifikationsreaktion, insbesondere die PCR verwendet wurde, entsteht ein sequenzidentisches Amplifikat, was allerdings nur ein Teilabschnitt der genomischen DNA darstellt. Werden hierbei ins besondere modifizierte dNTPs verwendet, dann ist das Amplifikat nicht molekular identisch, sondern nur sequenzidentisch zur ursprünglichen DNA. Werden bevorzugt mittels einer "Linkage- PCR" Teilabschnitte einzelner Gene zusammengefügt, dann leitet sich zum Beispiel das Amplifikat von mehreren Probenfragmenten ab. Das Amplifikat kann vorteilhafterweise auch eine zum ursprünglichen Molekül abweichende Molekülklasse aufweisen. All diese Produkte oder Abbilder sind Amplifikate im Sinne der Erfindung.

Es ist bevorzugt, dass ein Amplifikat amplifiziert wird und somit weitere Amplifikate erzeugt werden. Auf diese Weise kann eine besonders gute Sättigung der Fängermoleküle erreicht werden, sodass für nachfolgende Analysen eine hohe Anzahl an gebundenen Amplifikaten vorliegt.

Im Sinne der Erfindung bezeichnet PCR eine Polymerase-Kettenreaktion ("polymerase chain reaction"), einer vorzugsweise temperaturgesteuerten, enzymatischen Reaktion, die aufgrund einer vorliegenden DNA (Probenfragment) ein Amplifikat derselben herstellen kann. Der Amplifikationsschritt im erfindungsgemäßen Verfahren ist bevorzugt eine PCR.

Die Amplifikate werden in ihrem Kompartiment an den Fängermolekülen immobilisiert, sodass es auch nach der Reaktion zu keiner Vermischung der Amplifikate kommen kann. Dies ermöglicht eine anschließende ein-eindeutige Zuordnung von Amplifikat und entsprechender Probe.

Durch die Erfindung ist es somit möglich, eine Vielzahl von Probefragmenten zu amplifizieren, wobei jedes Fragment räumlich getrennt amplifiziert werden kann und die Trennung bis zur Produktebene (=Amplifikatebene) aufrechterhalten werden kann.

Es ist besonders bevorzugt, dass es sich bei den Kompartimenten um Kavitäten handelt. Es war völlig überraschend, dass die Einbringung von Proben in Kavitäten im Vergleich zu planaren Oberflächen vorteilhafte Effekte erzielen kann. So konnte der Probenverlust beim Aufbringen deutlich reduziert werden. Auch kam es zu signifikant weniger Kontamination. Zusätzlich wird ein Vermischen der Probenfragmente oder der entstehenden Amplifikate durch die Kavitäten komplett verhindert, was bei planaren Oberflächen eine weitere Fehlerquelle darstellt. Durch die verbesserte Handhabung welche durch die Kavitäten erzielt wird, kann das Verfahren als Massenverfahren eingesetzt werden.

Die Kavitäten der befüllten Festphase, bevorzugt des befüllten Multiwell-Arrays, werden beispielsweise durch unterschiedliche Methoden kompartimentiert und insbesondere voneinander isoliert. Jede Methode für die Kompartimentierung ist so beschaffen, dass es eine gewünschte Reaktion ermöglicht und ungewünschte Reaktionen oder Inhibitionen unterbindet.

Es war völlig überraschend, dass es mit dem Verfahren möglich ist, in den einzelnen Kompartimenten eine eigenständige biochemische und/oder chemische Reaktion durchzuführen. Hierdurch sind nicht nur High-Throughput Verfahren einfach und effizient durchführbar, sondern auch die Kontaminationsgefahr wird maßgeblich minimiert.

Die Anbindung bzw. Immobilisierung der Amplifikate an die Oberfläche der jeweiligen Mikrokompartimenten während der Amplifikationsreaktion ermöglicht es, die Amplifikate in nachfolgenden Reaktionen oder Analysen weiterhin voneinander getrennt zu halten. Dies ist von Wichtigkeit, wenn insbesondere die Mikrokompartimente geöffnet werden, um eine weitere Analyse oder Derivatisierung der Amplifikate (zum Beispiel eine Sequenzierung) durchzuführen. Zudem kann vorteilhafterweise ausgewählt werden, welcher Bereich der Amplifikate angebunden wird, z.B. bei DNA der sense oder der antisense Strang oder beide. Durch das Verfahren können die Herstellungskosten, sowie die Verbrauchskosten erheblich reduziert werden, da z. B. die Kosten für DNA-Beads (Capture-Beads), Magnetische Beads (Enrichment-Beads benötigt zur Aufreinigung nach der emPCR) und Chemikalien (Detergenzien, Puffer, Emulsionsöl, emPCR Reagenzien) eingespart werden. Weiterhin ist vorteilhaft, dass die Prozessführung vereinfacht ist. Die Durchführung der einzelnen Prozessschritte kann von weniger geschultem Personal durchgeführt werden, es wird keine spezielle, zu erlernende Expertise, wie bei den im Stand der Technik beschriebenen Verfahren zur Durchführung der Amplifikation und der Aufreinigung der Amplifikate mehr benötigt. Ein weiterer Vorteil des Verfahrens ist, dass Standard-Laborgeräte wie beispielsweise Inkubator, Heiz-/Kühlvorrichtung, Zentrifuge oder Vakuumsupport und Pipetten benötigt werden. Teure und wartungsintensive Spezialgeräte wie beispielsweise ein Coultercounter entfallen. Das Verfahren kann als ausgesprochen einfach, effizient und nicht fehleranfällig beschrieben werden.

Als weiterer Vorteil gegenüber dem Stand der Technik ist die schnellere Prozesszeit zu nennen. Es wird weniger Zeit benötigt, um z. B. eine Amplifikation durchzuführen. Beispielweise ist es mit einer bevorzugten Ausführungsform des Verfahrens möglich, Sequenzierchips vorzugsweise inklusive aller Arbeitsschritte wie Ansetzen des Reaktionsmixes, Einbringung von Probenfragmenten und Amplifikationsmix, Abdeckung, Durchführung der Reaktion und Waschen nach der Reaktion in bevorzugt 5 Stunden bereitzustellen, wohingegen die im Stand der Technik offenbarten Verfahren mehr als 11 Stunden benötigen. Außerdem kann eine höhere Effizienz und längere Amplifikate erreicht werden. So ist es mit einer bevorzugten Ausführungsform des Verfahrens möglich, sehr lange Amplifikate von mehr als 10.000 Basen (Nukleinsäurebasen) auf einer mikrostrukturieren Festphase zu immobilisieren. Besonders hervorzuheben ist die Amplifizierung und Immobilisierung von Probenfragmenten mit Längen über 500 Basenpaaren. Die Verfahren des Stands der Technik, wie beispielsweise die emPCR, sind aufgrund längerer Zyklenzeiten stark limitiert, was die Gefahr einer Tropfenfusionierung und somit einer Prozessverlangsamung, bzw. -inhibition erhöht.

Die Erfindung kann als Kombinationserfindung angesehen werden. Die Kombination von den bekannten Elementen in eine mikrostrukturierte Festphase führt zu überraschenden Effekten bei der Amplifikation von Probenfragmenten. So war es beispielsweise völlig überraschend, dass das Verfahren zur Amplifikation von zahlreichen Molekülen genutzt werden kann und es so universell einsetzbar ist. Außerdem kann das Verfahren schnell und einfach manuell oder automatisch durchgeführt werden. Erst die Kombination der einzelnen Verfahrensschritte, die derart nicht aus dem Stand der Technik bekannt sind, führt zu synergistischen Vorteilen, die eine effiziente Amplifikation und Immobilisierung von Molekülen bewirkt.

In einer weiteren bevorzugten Ausführungsform werden die Amplifikate im Anschluss an Schritt f) sequenziert und analysiert und/oder derivatisiert. Durch die räumliche Trennung der Amplifikate sowie der eindeutigen Zuordnung zu der entsprechenden Probe, können eine Vielzahl von Analysen an das Verfahren anschließen. Die Sequenzierung ist dabei besonders vorteilhaft. Auch die Herstellung von Derivaten, beispielsweise Proteinen ist eine bevorzugte Ausführungsform.

Bevorzugt ist, dass die Fängermoleküle kurze Nukleinsäuresequenzen umfassen. In einer besonders bevorzugten Ausführungsform sind die Fängermoleküle Primer, ganz besonders bevorzugt Primer, die für den Amplifikationschritt verwendet werden. So kann neben den Reaktionsprimern welche im Reaktionsmix enthalten sind eine weiter Primergruppe als Fängermoleküle verwendet werden. Besonders vorteilhaft ist, dass mindestens einer der Reaktionsprimer als Fängermoleküle zum Einsatz kommt. Diese Primer werden dann an die Festphase immobilisiert, sodass die Amplifikationsreaktion direkt an dem immobilisierten Primer (Fängermolekül) stattfindet. D.h. in dieser Ausführungsform werden sowohl die Probefragmente als auch die Amplifikate von den Fängermolekülen gebunden. Hierdurch können unter anderem Kosten und Zeit gespart werden. Außerdem steigt die Bindungsdichte, was die späteren Analysen erleichtert.

Bevorzugt ist außerdem, dass Verfahren die Probefragmente und/oder Amplifikate über kovalente Bindungen an die Fängermoleküle immobilisiert werden. Dies gewährleistet, dass die Bindung z.B. auch in der Elongationsphase der Amplifikation erhalten bleibt und sich nicht bei höheren Temperaturen wieder löst. Für gebundene Amplifikate bedeutet diese Ausführungsform zusätzlich, dass diese stabiler gebunden und die Bindung dadurch strapazierfähiger ist, was nachfolgende Analysen oder Reaktionen erleichtert.

In einer weiteren bevorzugten Ausführungsform betrifft die Erfindung das Verfahren, wobei die Einbringung des Probenfragments und die Einbringung des Reaktionsmixes in einem Schritt erfolgen, bevorzugt indem das Probenfragment vor der Einbringung mit dem Reaktionsmix vermischt wird. Je nach Aufbau der Festphase kann durch diese Vorgehen weitere Zeit gespart werden.

Ein Reaktionsmix bezeichnet im Sinne der Erfindung insbesondere die Gesamtheit aller Moleküle, die es erlauben, von einem Probenfragment Amplifikate herzustellen. Die Zusammensetzung des Mix legt in eindeutiger Weise fest, welche Probenfragmente in welcher Weise amplifiziert werden. Ein Reaktionsmix für RNA wäre beispielsweise ein Mix welcher eine RNAPolymerase enthält. Ein Reaktionsmix ist bevorzugt ein Amplifikationsmix.

Außerdem ist bevorzugt, dass der Reaktionsmix und/oder das Probenfragment über Zentrifugation, Vakuum, Zerstäuben, Eintauchen, Dipcoaten und/oder Bestreichen der Oberflächen der Festphase zugesetzt wird. Diese Methoden eignen sich besonders gut, da die Befüllung zeitsparend erfolgt und eine hohe Genauigkeit gewährleistet wird.

In einer bevorzugten Ausführungsform ist die mikrostrukturierte Festphase eine Oberfläche mit mindestens einem Einzelkompartiment, bevorzugt einer Vielzahl voneinander getrennter Einzelkompartimente. Die Festphase kann insbesondere ein Multiwell-Array sein. Ein Multiwell-Array bezeichnet im Sinne der Erfindung insbesondere eine räumliche Anordnung von Mikrokompartimenten, bevorzugt Kavitäten. In die Kavitäten kann ein Reaktionsmix eingefüllt werden, wobei innerhalb eines Mikrokompartimentes eigenständige Amplifikationsreaktionen ablaufen können. Es ist bevorzugt, dass zwischen den Mikrokompartimenten eines Multiwell-Arrays kein Austausch von Amplifikaten und/oder Probenfragmenten erfolgt.

Es kann bevorzugt sein, dass das Verfahren zur Bereitstellung von Amplifikaten für einen Sequenzierchip verwendet wird, wobei die Festphase insbesondere als ein Multiwell-Array bevorzugt aus einer regelmäßigen, einseitig strukturierten Anordnung aus Glasfasern ausgestaltet ist. Weiterhin können die Kavitäten vorteilhafterweise aus einem Multilagenverbund oder "track etched" Membranen bestehen. Das Multiwell-Array weist vorzugsweise 1 - 1000 Kavitäten mit Einzelvolumina zwischen 1 fL bis 1 mL auf. Das Multiwell-Array kann vorzugsweise ein Multifaserarray sein, dessen Grundeinheit optische Fasern sind. Diese optischen Fasern sind beispielsweise optische Glasfaserbündel, die es vorteilhafterweise ermöglichen, das bevorzugte Verfahren mit hohen Durchlaufgeschwindigkeiten durchzuführen.

Multiwell-Array bezeichnet insbesondere eine mikrostrukturierte Festphase, bevorzugt eine Oberfläche die verschließbare Vertiefungen enthält. Mutiwell-Array umfasst zudem einen Chip, welcher Kavitäten enthält.

Das Multiwell-Array weist bevorzugt eine regelmäßige Anordnung von hexagonalen Kavitäten auf. Es hat sich herausgestellt, dass hexagonale Kavitäten insbesondere für die Automatisierung des Verfahrens vorteilhaft sind und hierdurch die Kavitäten schneller gefüllt werden können.

Die gesamte oder Bereiche der Kavität oder nur die Wände der Kavitäten sind in einer bevorzugten Ausführungsform mit Metallen, Metalloxiden oder Kunststoffen beschichtet.

Metalle bezeichnen insbesondere chemische Elemente entsprechend der Nomenklatur des chemischen Periodensystems der Elemente (die sich im Gegensatz zu den Nichtmetallen im Periodensystem links der diagonalen Trennungslinie beginnend mit dem Element Beryllium (2. Gruppe) bis hin zum Polonium (16. Gruppe) befinden) sowie deren Legierungen und intermetallische Verbindungen mit charakteristischen metallischen Eigenschaften.

Alternativ oder zusätzlich kann mindestens eine Oberfläche mindestens einer Festphase mit nichtmetallischen Materialien beschichtet sein. Für nicht-metallische Materialien können in einer bevorzugten Ausführungsform Polymere verwendet werden. Polymere bezeichnen, nach einer Definition der IUPAC ("The International Union of Pure and Applied Chemistry") eine Substanz, die sich aus einem Kollektiv chemisch einheitlich aufgebauter, sich in der Regel aber hinsichtlich Polymerisationsgrad, Molmasse und Kettenlänge unterscheidender Makromoleküle (Polymermoleküle) zusammensetzt. Bei solchen sogenannten polymereinheitlichen Stoffen sind also alle Makromoleküle gleich aufgebaut und unterscheiden sich lediglich durch ihre Kettenlänge (Polymerisationsgrad). Man kann derartige Polymere als Polymerhomologe bezeichnen. Polymere können anorganische Polymere, voll- oder teilaromatischen Polymeren, Homopolymere, Copolymere, Biopolymere, chemisch modifizierte Polymere und/oder synthetische Polymere umfassen. Bevorzugte Polymere sind beispielsweise Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere oder Silikone. Polymere können aus der Gruppe umfassend anorganische Polymere, metallorganische Polymere voll- oder teilaromatischen Polymeren, Homopolymere, Copolymere, Biopolymere, chemisch modifizierte Polymere und/oder synthetische Polymere ausgewählt werden und umfassen Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere, Silikone, Proteine, DNA, RNA, Kohlenhydrate oder Polyhydroxxyalkanoate.

Es kann auch bevorzugt sein, eine Oberfläche mit Kohlenstoff insbesondere Karbon- oder Graphitfasern zu beschichten. Weiterhin kann auch bevorzugt sein, eine Beschichtung aus Kunststoff zu verwenden. Kunststoffe bezeichnen im Sinne der Erfindung insbesondere Materialien, deren wesentliche Bestandteile aus solchen makromolekularen organischen Verbindungen bestehen, die synthetisch oder durch Abwandlung von Naturprodukten entstehen.

Für die bevorzugte Beschichtung können Kunststoffe aus der Gruppe abgewandelter Naturstoffe, synthetische Kunststoffe (Polykondensate, Polymerisate, Polyaddukte) Duroplaste und/oder ungesättigte Polyesterharze verwendet werden.

Es kann bevorzugt sein, die Wände der Kavitäten mit Titan zu beschichten. Es war völlig überraschend, dass mittels einer Titanbeschichtung eine effizientere Bindung der Fängermoleküle erreicht werden kann. Außerdem bestehen aufgrund der vorteilhaften Oberflächeneigenschaften von Titan geringere Adhesionskräfte, wodurch weniger unspezifische Wechselwirkungen zwischen der Oberfläche und Reaktionsbestandteilen entstehen und auch Waschschritte schneller durchführbar sind. Weiterhin hat sich herausgestellt, dass mittels der Titanbeschichtung eine Unterbindung von optischem crosstalk bei der Sequenzierreaktion z. B. in dem System GS von Roche erreicht wird, bei denen Photonen beim Einbau von Nukleotiden freigesetzt werden und dann durch optische Fasern zum Detektor geleiten werden.

Die Oberfläche ist kann auch eine planare Fläche, die eine Strukturierung oder Beschichtung aus hydrophoben und/oder hydrophilen Bereichen enthält. Hydrophilie bedeutet im Sinne der Erfindung insbesondere wasserliebend, wohingegen Hydrophobie insbesondere wassermeidend bezeichnet. Die Oberfläche kann bereichsweise oder vollständig mit einer hydrophilen oder hydrophoben Beschichtung strukturiert sein. Hierbei kann ein hydrophober oder hydrophiler Stoff auf die Oberfläche aufgebracht sein. Hydrophobe Stoffe umfassen vorzugsweise Fette, Wachse oder Alkohole mit langen Alkylresten oder spezielle Coatings wie beispielsweise teflon carbon-black. Hydrophile Stoffe umfassen insbesondere Salze oder polare Verbindungen oder spezielle Coatings wie beispielsweise VISTEX.

Die Oberfläche kann in einer weiteren bevorzugten Ausführungsform eine 3-dimensionale Mikrostruktur sein, die einzelne Bereiche enthält, die durch geeignete Prozessführung voneinander zu Kompartimenten abtrennbar ist, z.B. kleine Säulen, an deren Spitze die Amplifikation abläuft. Es kann auch vorteilhaft sein, die Oberfläche planar und unstrukturiert auszugestalten, wobei sie mit einem Deckel oder einem sonstigen Verschlussmittel, wie beispielsweise einer Beschichtung, verschlossen wird, so dass die Kompartimente erzeugt werden.

Die Festphase, bevorzugt das Multiwell-Array, ist bevorzugt aus unterschiedlichen Materialien (halbleitenden, amorphen, kristallinen, faserartigen) gefertigt. Vorzugsweise können die Kavitäten unter anderem durch Ätzverfahren realisiert werden, wie zum Beispiel durch Trockenätzen/Reaktive Verfahren (unter anderem DRIE, Bosch-Prozess, ICP), nasschemische Verfahren (unter anderem Laugen, Säuren, HF) oder physikalischen Methoden (unter anderem Bohren, Sputtern, ionisches Ätzen). Die Festphase, bevorzugt das Multiwell-Array, kann bevorzugt beliebige Beschichtungen bevorzugt mit Metallen, Metalloxiden oder Kunststoffen enthalten. Die Kavitäten weisen bevorzugt eine unterschiedliche Geometrie auf, ausgewählt aus der Gruppe umfassend quadratisch, rechteckig, rund, oval, dreieckig, Hexagone, Oktagone, Polygone oder Kombinationen hiervon. Außerdem können die Kavitäten vorteilhafterweise unterschiedliche Tiefen bzw. Merkmalverhältnisse (insbesondere Abmessungen) aufweisen. Die Kavitäten sind vorteilhafterweise in unterschiedlichen Anordnungen zueinander (regelmäßig oder unregelmäßig) angeordnet.

Je nach Anzahl der in das Amplifikationssystem eingebrachten Probenfragmente werden in die Festphase, bevorzugt das Multiwell-Array, bevorzugt zwischen 1 und 1000 Fragmenten eingebracht. Weiterhin kann jede Kavität insbesondere zwischen 1 und 10 Fragmenten enthalten.

In einer weiteren bevorzugten Ausführungsform betrifft die Erfindung das Verfahren, wobei die Abdeckung und/oder Abtrennung der Kompartimente Öl, elektrische Kräfte, Phasenübergänge, inerte Bereiche und/oder Deckel umfasst. Besonders bevorzugt wird ein einziger Deckel verwendet um alle Kavitäten einer Festphase abzudecken. Diese Ausführungsform ist vorteilhaft, da die Handhabung erleichtert wird und so ein schnelles und effektives Abdecken aller Kavitäten erfolgt.

Der Deckel kann beispielsweise aus unterschiedlichen Materialien wie zum Beispiel Glas oder Polymeren bestehen, insbesondere aus Polydimethylsiloxan (PDMS), Polypropylen (PP), cyclic olefin copolymers (COC) oder cyclic olefin polymers (COP). Der Deckel kann nach der Befüllung mit Reaktionsmix oder zusammen mit dem Reaktionsmix auf das Multiwell-Array aufgebracht werden. Weiterhin kann dieser senkrecht aufgedrückt werden, oder waagerecht über das Multiwell-Array gezogen werden (slippen).Die Abdeckung weist Fängermoleküle auf.

Beim Verschließen der befüllten Kavitäten muss sichergestellt sein, dass die Amplifikate den Amplifikationsbereich nicht verlassen können. Bei der Verwendung eines Deckels wird rein mechanisch abgedichtet und die einzelnen Kavitäten voneinander fluidisch isoliert und somit einzelne Kompartimente für eine Amplifikationsreaktion erzeugt. Das Material des Deckels wirkt nicht reaktionsinhibierend und ist kompatibel zu den Amplifikationsreaktionen.

Der Deckel ist ebenfalls mit Fängermolekülen beschichtet. Diese Ausführungsform führte zu besonderen überraschenden Ergebnissen. So ist es dadurch möglich, nach Abschluss der Reaktion den Deckel von der Festphase zu trennen und separat zu untersuchen. Auch auf dem Deckel können so die Amplifikate ein-eindeutig den entsprechenden Proben zugeordnet werden.

Es ist bevorzugt, dass der Deckel die gleichen oder abgeänderte Fängermoleküle aufweist, um somit die Probenfragmente, Amplifikate oder Derivate der Amplifikate zu binden. Ist der Deckel mit anderen Fängermolekülen ausgestattet als die Festphase, so können im Deckel andere Moleküle immobilisiert werden, was je nach Anwendungsgebiet vorteilhafte Eigenschaften mit sich bringt.

Die Festphase, bevorzugt das Multiwell-Array, kann vorteilhafterweise unterschiedliche geometrische Ausgestaltungsformen besitzen. Es ist bevorzugt, dass die Kavitäten durch einen Deckel oder ein Verschlussmittel oder -element oder Abdeckung verschlossen und so voneinander räumlich getrennt werden. Im Sinne der Erfindung werden die Begriffe Verschlussmittel, -element und Abdeckung synonym verwendet. Es kann jedoch auch vorteilhaft sein, als Verschlussmittel eine Flüssigkeit, wie beispielsweise Öl zu verwenden. Dem Öl können bevorzugt Detergenzien zugesetzt sein.

Die Fängermoleküle können entweder direkt oder indirekt an der Festphase immobilisiert sein. Dabei kann das Fängermolekül, bevorzugt eine Nukleinsäure, an einem oder mehreren Enden eine chemische Kopplungsgruppe oder -sequenz enthalten. Bevorzugt ist eine Kopplungsgruppe -sequenz am 5'-Ende angebracht. Die Kopplungsgruppe oder - sequenz kann z.B.. Biotin, Double-Biotin, NH₂, SH, Acrydite und/oder Polynukleotidsequenzen sein.

Außerdem ist bevorzugt, dass die Fängermoleküle mindestens ein Spacermolekül aufweisen. Diese Spacer ist bevorzugt zwischen der Kopplungsgruppe und der Nukleinsäuresequenz angeordnet.

Die Länge des Fängermoleküls ist bevorzugt nicht limitiert, liegt aber bevorzugt zwischen 1 und 1.000 Einzelmolekülen, im Fall von DNA 1 bis 1000 Basen. Diese Längen liefern besonders gute Ergebnisse bezüglich der Bindungsstabilität und -spezifität.

In einer weiteren bevorzugten Ausführungsform betrifft die Erfindung das Verfahren, wobei die Fängermoleküle mindestens eine Anbindestelle für Probenfragmente und/oder Amplifikate umfassen und.

Es ist außerdem bevorzugt, dass die Fängermoleküle und/oder die Amplifikate eine photoaktivierbare Spaltgruppe aufweisen. Die photoaktivierbare Gruppe kann z.B. Caged-Verbindungen wie photolabiles Nitrophenyl-Ethyl, 2-Nitrobenzyl Derivate, Coumarinyl-Derivate, p-Hydroxyphenacyl-Derivate, Benzoin-Derivate, 1-Acyl-7-Nitroindolin-Derivate sein. Über die photoaktivierbare Gruppe können die Fängermoleküle an einer Oberfläche immobilisiert werden. Die photoaktivierbare Gruppe wird unter Lichteinfall gespalten oder chemisch so verändert, dass die über die Fängermoleküle immobilisierten Amplifikate gezielt von der Oberfläche entkoppelt werden und für Analysen zur Verfügung stehen.

Besonders bevorzugt ist, dass die Fängermoleküle eine Promotorbindestelle für eine RNA-Polymerase oder ein zellfreies Expressionssystem enthalten.

In einer weiteren bevorzugten Ausführungsform betrifft die Erfindung ein Verfahren, wobei die Fängermoleküle ein Spacermolekül aufweisen, an einem oder mehreren Enden eine chemische Kopplungsgruppe enthalten und/oder hiermit entweder direkt oder indirekt an der mikrostrukturierten Festphase immobilisiert sind. Die Fängermoleküle sind vorzugsweise kurze Nukleinsäuresequenzen. Vorteilhafterweise ist ein Spacermolekül zwischen der Kopplungsgruppe und Nukleinsäuresequenz vorhanden. Über die Einbringung eines Spacers zwischen Kopplungsgruppe und Nukleinsäuresequenz wird ein physikalischer Abstand zwischen Oberfläche und Nukleinsäure erzeugt. Der Spacer kann unter anderem aus einer Kohlenstoffkette oder einer Polynukleotidsequenz (Poly-T Sequenz) bestehen.

Die Fängermoleküle enthalten bevorzugt für die orientierte Anbindung an einer Oberfläche an einem oder mehreren Enden eine chemische Kopplungsgruppe oder Kopplungssequenz am beispielsweise 5' Ende der Nukleinsäure. Diese Gruppe ist ausgewählt aus der Gruppe umfassen Biotin, Doppel-Biotin, Amino-Gruppe (NH2), Thiol-Gruppe(SH), Acryditen, Polynukleotidsequenz, Streptavidin, His-Tag (His = Histidin), Nickel-NTA (NTA = Nitrilotriessigsäure), myc- oder flag-Tag oder Kombinationen hiervon.

Die Fängermoleküle sind bevorzugt direkt oder indirekt immobilisiert. Verfahren für die direkte Immobilisierung von Nukleinsäuren umfassen unter anderem Hydrogele, UV-Crosslinking, Kopolymerisation in Acrylamidgele, Affinitätsbindungen (avidinstreptavidin), ionische, absorptive oder nicht-kovalente Bindungen. Verfahren für indirekte Immobilisierung verwenden Linkermoleküle, vorzugsweise homobifunktionale oder heterobifunktionale Linkermoleküle, um die Fängermoleküle bevorzugt an der Oberfläche der Kavitäten der Festphase zu immobilisieren. Substanzen für die Immobilisierung umfassen beispielsweise N, N'-Diisopropylcarbodiimid (DIC), N-Hydroxysuccinimid (NHS), Melamido, NTMTA, N-(3-Trifluoroethanesulfonyloxypropyl) anthrachinon-2-carboxamid (NTPAC), 4 -(Nmaleimidomethyl) cyclohexan-1-carbonsäure 3 - sulfo-n-hydroxysuccinimide ester sulfo-nhydroxysuccinimidester (Sulfo-SMCC), Glycidyloxipropyltrimethoxysilan (GOPTS), Trimethoxysilyil propylmethacrylat (MPTS), Methanethiosulfonat (MTS), 1-Ethyl-3-(3-dimethylaminopropyl) carbodiimid (EDC), Glutaraldehyde, N, N'- Dicyclohexylcarbodiimid (DCC), Acrydite, silanized nucleic acids, phosphorothioate. Der homobifunktionale Linker ist bevorzugt 1,4-phenylene diisothiocyanate (PDITC) und erreicht insbesondere, die Wände der Kavitäten mit Fängermolekülen mit Sequenzlängen zwischen 10 und 500 zu beschichten. Die Anbindung der Fängermoleküle an eine Festphase ist bevorzugt kompatibel zu Amplifikationsreaktionen und eventuellen nachfolgenden Derivatisierungen. Das heißt, die chemische, physikalische oder biologische Anbindung der Fängermoleküle an die Oberfläche oder einen Bereich dieser, hat keine hemmende Wirkung auf nachfolgende Reaktionen.

Es ist bevorzugt, dass die Fängermoleküle mindestens eine Anbindestelle für Probenfragmente und/oder Amplifikate umfassen und vorzugsweise eine Länge von 1 bis 1000 Einzelmolekülen aufweisen. Im Sinne der Erfindung bezeichnen Fängermoleküle insbesondere molekulare Einheiten, die an eine Festphase angebunden sind und die Anlagerung von amplifizierten Probenfragmenten erlauben oder Amplifikate binden. Es war völlig überraschend, dass Fängermoleküle bereitgestellt werden konnten, die spezifisch die Amplifikate binden und immobilisieren. Hierdurch wird die Aufreinigung der Amplifikate erheblich vereinfacht, wobei die Amplifikate für weitere Reaktionen an den Fängermolekülen zur Verfügung stehen.

Es ist bevorzugt, dass die Probenfragmente an mindestens einem Ende Adaptoren mit einer einheitlichen Sequenz aufweisen. Die bevorzugte Länge der Adaptoren liegt bei bis zu 1000 Nukleotiden. Die Adaptoren können unterschiedliche funktionelle Einheiten aufweisen. Adaptoren sind bevorzugt am Anfang und Ende eines Probenfragments in Form von einheitlichen und eindeutigen Sequenzen vorhanden. Sie definieren diej enigen Bereiche der Probenfragmente, die amplifiziert werden. Die Adaptoren fungieren insbesondere für eine PCR als Primer. Einer der beiden Adaptoren ist vorteilhafterweise ganz oder zu einem Teil identisch zur Sequenz des Fängermoleküls. Bei Systemen für die Sequenzierung verfügt die zu sequenzierende DNA über zwei definierte und meist vom Hersteller des Systems vorgegebene Adaptoren. Mittels der Adaptoren können entweder alle oder nur bestimmten Untereinheiten der Gesamtheit aller Probenfragmente amplifiziert werden. Weiterhin können die Adaptoren vorzugsweise mehrere unterschiedliche funktionelle Einheiten hintereinander besitzen, die insbesondere Bindestellen für RNA-Polymerasen oder zellfreie Expressionssysteme sein können. Die Adaptorsequenzen können beispielsweise aus gängigen Sequenziersystemen stammen (unter anderem Applied Biosystems/Life Technologies, Ion Torrent/Life Technology, Roche, Illumina, George Church/Dover Systems) und hierbei sequenzidentisch sein oder weitere Sequenzen oder Modifizierungen enthalten. Damit können die Adaptorsequenzen eine sequenzierfähige Oberfläche schaffen. Die Oberfläche ist sequenzierfähig, wenn an die Fängermoleküle amplifizierte Probenfragmente gebunden haben. Die Amplifikate stehen für weitere Untersuchungen zur Verfügung.

Die Promoterbindestelle verlängert den Adaptor entweder an seinem einen oder anderen Ende oder an und/oder in einer beliebigen Stelle des Fängermoleküls. Die Sequenz der Fängermoleküle kann vorteilhafterweise mit der von Sequenziersystemen identisch oder teilidentisch sein, wie beispielsweise mit denen von Applied Biosystems/Life Technologies, Ion Torrent/Life Technology, Roche, Illumina, George Church/Dover Systems. Die Sequenz des Fängermoleküls stellt bevorzugt eine Anbindestelle für Probenfragmente dar, insbesondere vermittelt durch die Affinität eines der Adaptoren zu dem Fängermolekül. Diese Sequenz verlängert den Adaptor vorzugsweise am 3'- oder 5' Ende. Die Adaptoren können eine oder mehrere zusätzliche Sequenzen an einem oder beiden Enden enthalten, sodass während der Reaktion die Probenfragmente um diese speziellen Sequenzen verlängert werden.

In einer bevorzugten Ausführungsform umfassen die Probenfragemente bevorzugt DNA oder RNA. Ein Probenfragment ist bevorzugt ein Molekül, das amplifiziert werden soll. Die Gesamtheit aller Probenfragmente bildet die Probe ab. Vorzugsweise handelt es sich bei den Probenfragmenten um Fragmente genomischer DNA, aber auch cDNA oder RNA. Die Fragmente können beispielsweise, wie bei der Next Generation Sequenzierung durch Scherung genomischer DNA erzeugt werden. Die Gesamtheit der Probenfragmente besteht bevorzugt aus einzel und/oder doppelsträngigen Nukleinsäuresequenzen (1 - 10¹⁰ Basenpaare (bp)), wobei jedes Probenfragment bevorzugt an den beiden Enden eine oder mehrere Adaptorsequenzen (1 bp - 1000 bp) enthält. Die Probenfragmente können vorzugsweise direkt oder indirekt über ein eingebautes Label aus DNA oder RNA amplifiziert oder derivatisiert werden. Es ist bevorzugt, dass die Probenfragemente in einer Länge von 1 bis 10¹⁰, bevorzugt 1 - 10⁸ Einzelmolekülen einzelsträngig oder doppelsträngig vorliegen. Die Probenfragmente können unter anderem aus Viren, Pro- oder Eukaryoten gewonnen werden oder synthetischer Natur sein. Die Länge einer Probe kann vorzugsweise zwischen 1 und 10¹⁰ Einzelmonomeren betragen. Solche Proben können nach verschiedenen Verfahren in einzelne Fragmente unterteilt werden. Die Fragmente können einzel- oder doppelsträngig sein.

In einer bevorzugten Ausführungsform ist der Amplifikationsschritt insbesondere ausgewählt aus der Gruppe umfassend PCR, RPA, RCA, SDA, NASBA oder LAMP Dabei haben die Abkürzungen insbesondere die folgende Bedeutung: PCR steht für polymerase chain reaction, RPA 5 steht für Recombinase-Polymerase-Amplification, RCA steht für rolling circle amlification, SDA steht für Strand Displacement Amplification, NASBA steht für Nucleic acid sequence based amplification, LAMP steht für loopmediated isothermal amplification. Die Erfindung ist dabei jedoch nicht auf diese Amplifikationsmöglichkeiten beschränkt.

Der Reaktionsmix umfasst bevorzugt Einzelbausteine für die Synthese der Amplifikate, ein Energiesystem, ein Synthesesystem und 1 bis 10⁶ verschiedene Adaptorsequenzen. Der Reaktionsmix kann insbesondere auch ein handelsüblicher PCR Amplifikationsmix sein. Der Amplifikationsschritt stellt vorzugsweise ein isothermes Amplifikationssystem dar. Es kann bevorzugt sein, dass mit einem oder mehreren Amplifikationssystemen Probenfragmente amplifiziert und beispielsweise in den Kavitäten immobilisiert werden. Nach der Reaktion wird der Multiwell-Array beispielsweise mit Natriumhydroxid (NaOH) denaturierend gewaschen, um ungebundene Reaktionsprodukte zu entfernen und Kontamination zu vermeiden. Der Reaktionsmix enthält vorzugsweise eine DNA-Polymerase, insbesondere eine Pfu oder Taq Polymerase und dNTPs oder ein Mix aus dATPs, dCTPs, dGTPs, dTTPs und dUTPs und insbesondere ein UNG-Verdausystem. Weiterhin sind bevorzugt ein bis vier Adaptoren enthalten, die eine Sequenzlänge zwischen 10 und 500 Nukleotiden aufweisen.

Das Molekül dNTP (Desoxyribonukleosidtriphosphat) steht im Sinne der Erfindung insbesondere für molekulare Monomere, die für die Synthese eines DNA-Stranges mittels eines Amplifikationssystems benötigt werden. Es kann bevorzugt sein, dass als Probenfragment RNA zur Amplifikation vorliegt, so dass insbesondere eine RNA Polymerase und anstelle der dNTPs umfassend dATP (Desoxyadenosintriphosphat), dGTP (Desoxyguanosintriphosphat), dTTP (Desoxythymidintriphosphat), dCTP (Desoxycytidintriphosphat) die Moleküle dATP (Desoxyadenosintriphosphat), dGTP (Desoxyguanosintriphosphat), dUTP (Desoxyuridintriphosphat), dCTP (Desoxycytidintriphosphat) verwendet werden.

Es war völlig überraschend, dass es mit dem bevorzugten Verfahren möglich ist, eine Amplifikationsreaktion durchzuführen und insbesondere gleichzeitig die generierten Amplifikate zu immobilisieren. Es ist dabei bevorzugt, dass ein Amplifikationsschritt 1 bis 10¹⁰ Amplifikate generiert.

In einer bevorzugten Ausführungsform umfasst der Reaktionsmix reaktionsverbessernde Komponenten. Reaktionsverbessernde Komponenten umfassen vorzugsweise bovine serum albumin (BSA), Agarose, Tween, ultrageschallte Nukleinsäuren und/oder ein UNG Verdau System.

Vorteilhafterweise ist die Amplifikation keine emPCR. Die Generierung von Amplifikaten basierend auf einem Probenfragment und Anordnung der Amplifikate auf einer Oberfläche werden überraschenderweise miteinander verknüpft, so dass die emPCR komplett umgangen werden kann. Es war völlig überraschend, dass die Amplifikation von Molekülen in einer Festphase möglich ist, wobei die generierten Amplifikate von Fängermolekülen gebunden werden. Die beiden Schritte, Einbringung einer fragmentierten Probe in eine räumlich definierte Anordnung von Mikrokompartimenten und Anbindung bzw. Immobilisierung der Amplifikate an die Oberfläche der jeweiligen Mikrokompartimenten während der Amplifikationsreaktion, um die Amplifikate in nachfolgenden Reaktionen oder Analysen weiterhin voneinander getrennt zu halten, werden von dem Verfahren kombiniert. Dadurch ergeben sich überraschende synergistische Effekte, wie Zeit- und Kostenvorteile. Die Erfindung weist zahlreiche Vorteile gegenüber der im Stand der Technik offenbarten Verfahren auf, so dass zeitintensive Amplifikationsreaktionen wie z. B. die emPCR substituiert werden können.

Weiterhin ermöglicht das Verfahren mikrostrukturierte Oberflächen ortsaufgelöst mit Amplifikaten von Probenfragmenten zu beschichten, um danach neben einer Sequenzierung, eine Derivatisierung oder weiteren Amplifikation durchzuführen und somit auch neue Produkte herzustellen, wie sie mit dem bisherigen Verfahren nicht zugänglich sind.

Die Erfindung betrifft zudem eine Vorrichtung für die Durchführung eines Verfahrens zur Amplifikation von Probenfragmenten. Eine Vorrichtung für die Durchführung einer Reaktion umfasst insbesondere eine Kassette, die beispielsweise ein Multiwell-Array aufnehmen kann und in einen Zustand bringt, der die enzymatische Reaktion der Amplifikation fördert. Hierzu zählt beispielsweise ein guter Wärmeeintrag, im Falle der Verwendung eines temperaturgesteuerten Amplifikationssystems. Die Vorrichtung besteht vorzugsweise aus einem Material, wie Kunststoff oder Metalle, welches eine gute Temperatursteuerung ermöglicht und wird in einem Gerät durchgeführt, was ebenfalls eine gute Temperatursteuerung ermöglicht.

Bevorzugt ist eine Vorrichtung für die Durchführung eines erfindungsgemäßen Verfahrens umfassend
g) eine Festphase in Form eine Pikotiterplatte mit mehreren Kompartimenten, wobei Fängermoleküle an die Oberfläche der Festphase, bevorzugt der Kompartimente gebunden sind und
h) eine Abdeckung, wobei Fängermoleküle an die Abdeckung gebunden sind.

Die Vorrichtung verhindert weiterhin eine Kontamination mit Fängermolekülen und Probenfragmenten, da die in der Kassette oder Multiwell-Array vorliegenden Kavitäten durch eine Abdeckung vollständig bedeckt werden, so dass eine zuverlässige mechanische Versiegelung der Kavitäten z. B. des Multiwell-Arrays besteht. Außerdem weist die Vorrichtung eine Befüllvorrichtung auf, die eine leichte Befüllung der einzelnen Kompartimente oder Kavitäten ermöglicht. Die Reaktionsvorrichtung kann mit einem PCR-Temperaturprotokoll beaufschlagt werden.

Die Vorrichtung kann insbesondere als ein Front-end Komplement für beispielsweise die Sequenziersysteme GS FLX, GS FLX Titanium, GS Junior (Roche) und das System PGM - Personal Genome Machine (Ion Torrent) bezeichnet werden.

Besonders vorteilhaft ist, dass Fängermoleküle an die Abdeckung gebunden sind. Eine solche Vorrichtung ist vorteilhaft, da durch ein Verfahren zwei Arrays hergestellt werden können.

In einer weiteren bevorzugten Ausführungsform betrifft die Erfindung insbesondere ein Masterarray, das mit dem erfindungsgemäßen Verfahren hergestellt wurde.

Vorteilhaft dabei ist, dass sich mit dem erfindungsgemäßen Verfahren wieder verwendbare Masterarrays für die Herstellung von DNA- RNA- und Proteinmikroarrays generieren lassen. Durch die Wahl der Probenfragmente und des Reaktionsmixes, der im Fall von Proteinmikroarrays auch als Derivatisierungsmix bezeichnet wird, besteht eine freie Wahl, welches Array erzeugt werden soll. Ein mit dem beschriebenen Verfahren hergestelltes Multiwell-Array, welches ortsaufgelöst immobilisierte Amplifikate an Probenfragmenten enthält, kann als Vorlage für eine Kopierreaktion dienen und die Herstellung weiterer Masterarrays wird ermöglicht.

Dadurch dass die Fängermoleküle nicht nur in der strukturierten Festphase sondern auch an dem Deckel, mit dem das Array während der Reaktion verschlossen ist, vorhanden sind, werden die Amplifkate unter Beibehaltung der räumlichen Verteilung an Deckel und Festphase immobilisiert. Über dieses Verfahren werden vorzugsweise zwei Masterarrays generiert. Erstens ein Multiwell-Array für die Durchführung von einer initialen Sequenzierreaktion, das danach für die Herstellung von DNA- RNA- und Proteinmikroarrays verwendet werden kann. Zweitens ein planares DNA-Masterarray, mit dem weitere DNA-Mikroarrays generiert werden können. Hierbei wird mittels einer Hybridisierungsreaktion ein Primer mit einem freien 3' Ende an die an der Oberfläche befindlichen Amplifikate angebunden und nachfolgend mittels eines Reaktionsmixes, vorzugsweise enthaltend eine Pfu-Polymerase, verlängert. Da der Primer an seinem 5' Ende eine reaktive Gruppe aufweist, kann das Array mit einer Fest- oder Flüssigphase in Kontakt gebracht werden, die mit den Gruppen reagiert. Nach der Reaktion und dem Entfernen der ausgehärteten Flüssigphase bzw. der Festphase unter denaturierenden Bedingungen enthält das initiale DNAMasterarray immer noch die einzelsträngige DNA, das zweite Substrat dagegen den von einem Amplifikationssystem erzeugten Gegenstrang. Mittels diesem Verfahren ist man in der Lage, qualitativ hochwertige DNA-Mikroarrays herzustellen, die gegenüber dem Stand der Technik sehr lange DNA Sequenzen enthalten können (vorzugsweise >> 200 Nukleotide), die qualitativ sehr hochwertig sind, da eine Pfu-Polymerase mit Proof-Reading Funktion nur ein falsches Nukleotid auf 10⁶ Nukleotide einbaut.

Das Masterarray kann bevorzugt zur Herstellung von DNA-, RNA- oder Proteinmikroarrays und/oder zur Sequenzierreaktion verwendet werden. Das Multiwell-Array bei dem in einigen oder mehreren Kavitäten Amplifikate eines Probenfragments vorhanden sind, kann für unterschiedliche Derivatisierungen, Analysen oder Reaktionen zur Verfügung stehen, zum Beispiel als wieder verwendbares Masterarray für die Herstellung von DNA- RNA- oder Proteinmikroarrays.

Weiterhin kann das Masterarray für eine Sequenzierreaktion dienen. Derivatisierung umfasst insbesondere den Abbau, die Erweiterung oder die Transformation von Amplifikaten oder weiteren Molekülen, die an die Amplifikate gebunden oder davon abgespalten werden und die nochmalige Amplifikation von Amplifikaten.

Das Masterarray kann bevorzugt zur gleichzeitigen Amplifikation unterschiedlicher Probenfragmente in separaten Kompartimenten verwendet werden. Die Probenfragmente werden bevorzugt entsprechend einer emPCR hergestellt/vorbereitet. Hierzu wird die zu untersuchenden/amplifizierenden Probe vorzugsweise fragmentiert und je ein Adaptor an die Enden jedes Probenfragments angekoppelt. Eine Festphasen-Oberfläche ist mit Fängermolekülen beschichtet und so strukturiert, dass durch eine geeignete Prozessführung diese Oberfläche in eine Vielzahl von voneinander getrennten und abgeschlossenen Einzelkompartimenten unterteilt ist. Vor dem Erzeugen oder Verschließen der Einzelkompartimente ist es bevorzugt, sowohl Probenfragmente als auch ein Reaktionsmix in die Einzelkompartimente einzubringen. Die Einbringung besagter Komponenten kann entweder gleichzeitig oder sequentiell erfolgen. In jedem Einzelkompartiment kann somit eine unabhängige Amplifikationsreaktion des Probenfragments durch den Reaktionsmix und den Amplifikationsschritt erfolgen. Es ist bevorzugt, dass während oder nach der Amplifikation das Amplifikat in Bereichen des Einzelkompartiments an Fängermoleküle immobilisiert wird. Dies erzeugt eine immobilisierte räumliche Verteilung der Amplifikate, die der ursprünglichen Verteilung der Probenfragmente entspricht.

Das erzeugte Verteilungsmuster wird im Sinne der Erfindung insbesondere als Masterarray bezeichnet. Das Masterarray steht für nachfolgende Derivatisierungen, Analysen oder Reaktionen zur Verfügung und kann im Besonderen zur Sequenzierung der Probenfragmente verwendet werden.

In einer weiteren bevorzugten Ausführungsform betrifft die Erfindung ein Kit zur Amplifikation von Nukleinsäuren umfassend
k) eine Festphase in Form einer Pikotiterplatte mit mehreren Kompartimenten, wobei Fängermoleküle an die Oberfläche der Festphase, bevorzugt der Kompartimente gebunden sind,
l) eine Abdeckung und
m) mindestens einen Reaktionsmix
wobei Fängermoleküle an die Abdeckung gebunden sind.

Es war völlig überraschend, dass ein Kit hergestellt werden konnte, mit dem sich das erfindungsgemäße Verfahren durchführen lässt. Es war dabei nicht zu erwarten, dass die Festphase mit den gebundenen Fängermolekülen dauerhaft gelagert werden kann, ohne dass die Fängermoleküle ihre Bindefunktion verlieren. Dies wurde erst durch das Einfügen der Kompartimente möglich. Das erfindungsgemäße Kit kann somit über einen langen Zeitraum gelagert werden und erleichtern daher die Durchführung des erfindungsgemäßen Verfahrens um ein Vielfaches.

### Beispiele

Die Erfindung wird nunmehr anhand eines Ausführungsbeispiels und den beigefügten Zeichnungen beispielhaft beschrieben. Bei den Beispielen und Abbildungen handelt es sich um bevorzugte Ausführungsvarianten, welche die Erfindung nicht beschränken. Die beschriebenen Vorteile konnten auch für die anderen genannten Ausführungsvarianten nachgewiesen werden und treffen nicht nur auf das konkrete Beispiel zu. Es zeigt:
Abb. 1 Ausschnitt eines Multiwell-Arrays
Abb. 2 Bevorzugte geometrische Ausgestaltungen von Kavitäten
Abb. 3 Weitere geometrische Ausgestaltungen von Kavitäten
Abb. 4 Bevorzugte Fängermoleküle mit ihrer Orientierung
Abb. 5 Bevorzugte Adaptoren
Abb. 6 Bevorzugte Vorrichtung
Abb. 7 Weitere bevorzugte Vorrichtung
Abb. 8 Bevorzugte Auswertung
Abb. 9 Beispielhafte Versuchsergebnis einer Festphasen-PCR
Abb. 10 Beispielhafte Versuchsergebnis mit Signalen von befüllten Kavitäten
Abb. 11 Deckel von Multiwell-Array nach der Reaktion 30

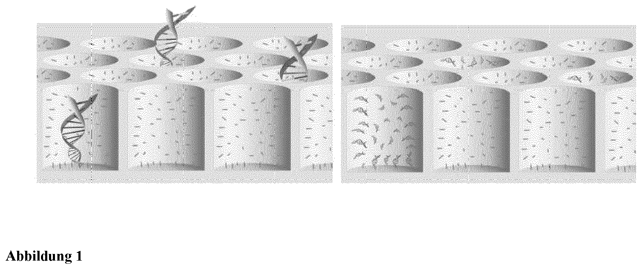

Abb. 1 zeigt einen schematischen Ausschnitt eines Multiwell-Arrays mit einigen Kavitäten. Alle Kavitäten sind mit Fängermolekülen beschichtet. Es wird ein Amplifikationsmix mit Probenfragmenten eingebracht (links) und die Kavitäten verschlossen. Nach der Amplifikation sind die jeweiligen Kavitäten mit Amplifikaten beschichtet (rechts).

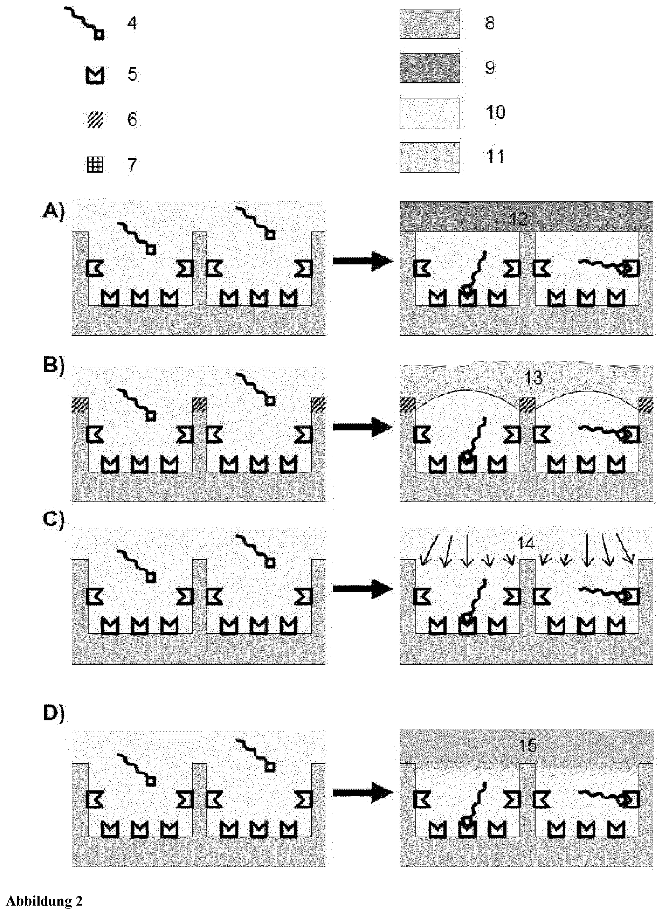

Abb. 2 zeigt eine mögliche Darstellung der unterschiedlichen geometrischen Ausgestaltungsformen zur Erzeugung der Kompartimentierung für die nachfolgende Amplifikation. Es wird jeweils der befüllte Grundzustand gezeigt und wie die einzelnen Bereiche gegeneinander abgeschirmt werden. Die Hauptauslegung sind Kavitäten (A) die mit einem Deckel verschlossen werden. Diese Kavitäten können aber auch mit Öl (B), Feldern, insbesondere elektrische, oder durch Temperatur (C) oder ein Phasenübergangsmaterial (D) voneinander getrennt werden.

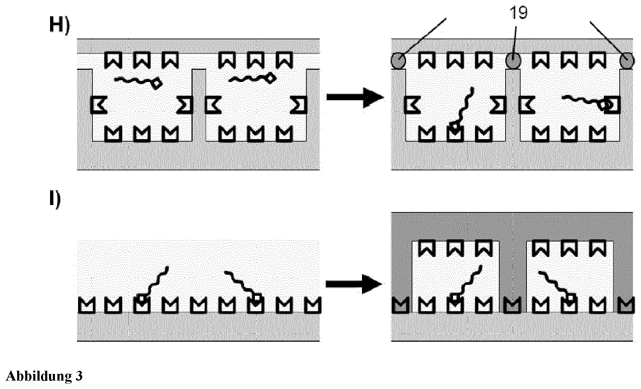

Abb. 3 zeigt ebenso wie Abb. 2 eine mögliche Darstellung der unterschiedlichen geometrischen Ausgestaltungsformen zur Erzeugung der Kompartimentierung für die nachfolgende Amplifikation. Weitere Auslegungen sind sämtliche Strukturen, die zu einer digitalen PCR fähig sind, die zusätzlich mit Fängermolekülen beschichtet werden (E). In einer invertierten Auslegungsform (F) werden die Probenfragmente zunächst auf eine planaren Oberfläche mit Fängermolekülen verbracht und dann mit einem Multiwell-Array verdeckelt. Dies ist eine Umstellung des Befüllvorganges, besitzt jedoch den Vorteil, dass man auch Proben mit stark verdünnten Probenfragmenten an der planaren Oberfläche anreichern kann. Eine direkte Befüllung könnte sonst aufgrund einer zu starken Verdünnung nicht durchführbar sein bzw. zu wenige Kavitäten mit einem Probenfragment befüllen. Die Kavität entsteht in dem Moment des Verschlusses. Version F kann unter anderem dazu verwendet werden, um DNA von Mikroarrays in ein Multiwell-Array zu kopieren.

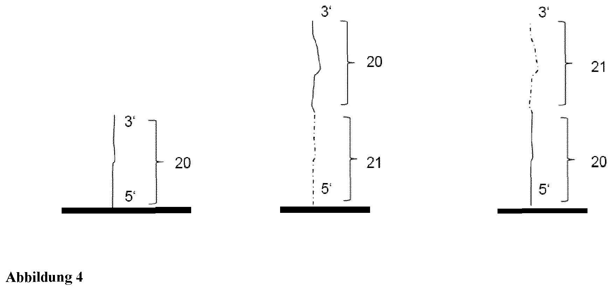

Abb. 4 zeigt Unterschiedliche Möglichkeiten für Fängermoleküle mit ihrer Orientierung.

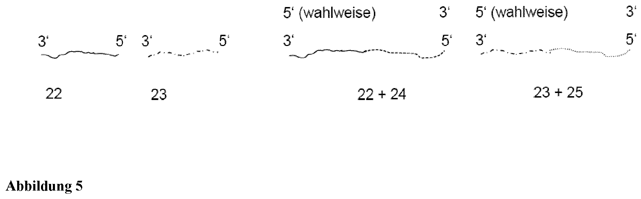

Abb. 5 zeigt mögliche Ausformungen der Adaptoren.

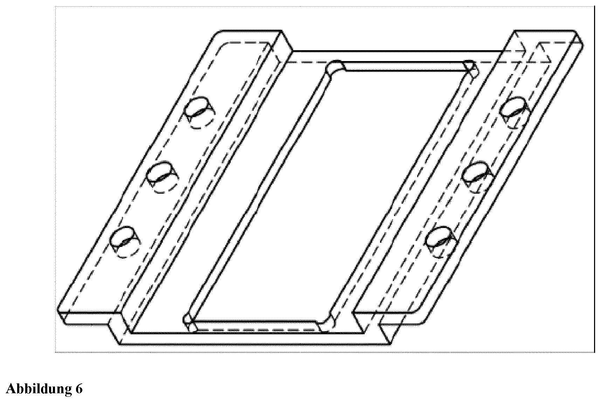

Abb. 6 zeigt eine mögliche Vorrichtung/Kassette für eine erfindungsgemäße Amplifikation und/oder Sequenzierung.

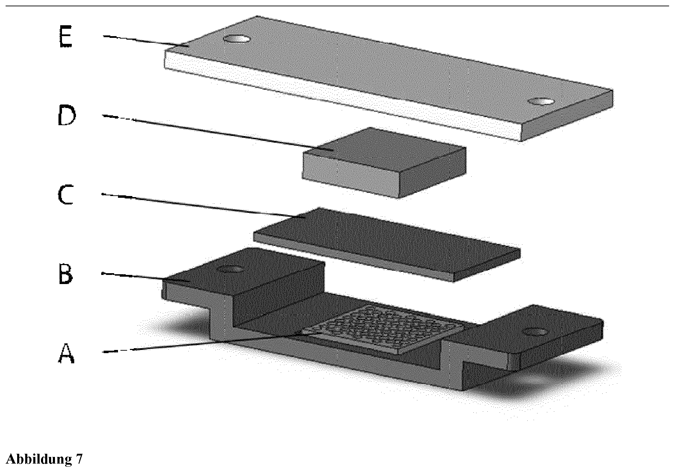

Abb. 7 zeigt eine Explosionszeichnung einer bevorzugten Vorrichtung für die Reaktion. Multiwell-Array (A) in der Vertiefung eines Kupferhalters (B), Deckel (C), 3 mm starker elastischer Stempel (D), Klemmdeckel (E). Um den Amplifikationsmix mittels Zentrifugation in das Array einzubringen, werden die Teile C bis D ersetzt durch eine Befüllvorrichtung die einen 150 µm hohen mikrofluidische Kammer realisiert.

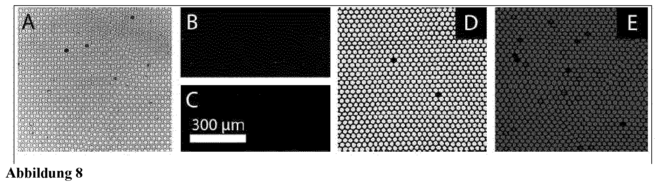

Abb. 8 zeigt eine mögliche Auswertung einer Reaktion in einem Multiwell-Array mit einem Well-Volumen von 19 Pikolitern. Bild (B) bis (E) zeigen die Scans von eingefärbten Deckeln nach der Reaktion. Bild (A) zeigt in Mikroskopbild eines Multiwell-Arrays. Bild (B) zeigt eine Spezifitätekontrolle. In diesem Fall ist eine Fängermolekül immobilisiert das nicht komplementär ist zur Sequenz des DNA Fragments, wobei keine positiven Signale generiert wurden. Bild (C) zeigt eine no-DNA Fragment Kontrolle. Auch hier werden keine Signale generiert. Dieses Ergebnis zeigt dass im Falle des Vorhandenseins eines DNA Fragments auch ein Signal generiert wird. Bild (D) zeigt die Signale eines Deckels bei dem eine Hybridizierung eingesetzt wurde um die Reaktion zu visualisieren. Dieses Ergebnis zeigt, dass an die Fängermoleküle tatsächlich auch die richtige Sequenz der DNA Fragmente gebunden wurde. Bei Bild (E) wurde das oberflächengebundene PCR-Produkt durch die Ankopplung von Streptavidin-Cy5 an die während der Reaktion eingebauten biotin-Moleküle angebunden und die Reaktion visualisiert.

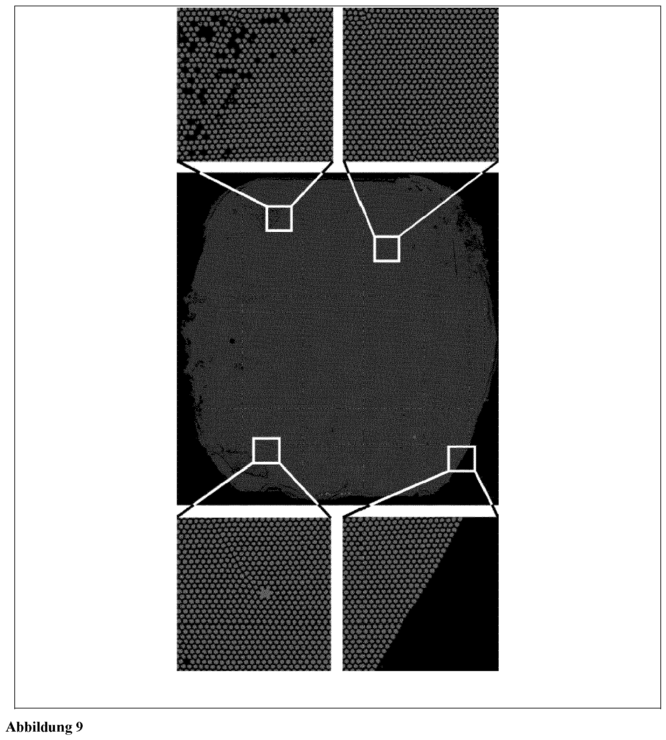

Abb. 9 zeigt eine mögliche Durchführung einer Reaktion in 110.000 19 Pikoliter Wells. In 99.18 % aller Wells (109.101 Signale) wurden die PCR Produkte an die Fängermoleküle mittels dem Mechanismus der Festphasen-PCR gebunden. Dieses Ergebnis zeigt, dass man in der Lage ist mit diesem System auch auf Flächen > 1 cm² die Reaktion durchzuführen.

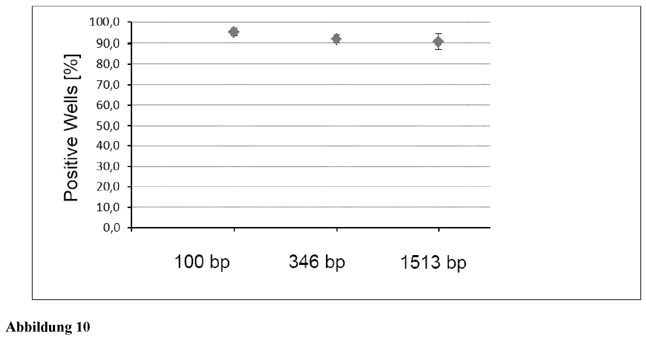

Abb. 10 zeigt beispielsweise einen Versuch, für den DNA Fragmente der Länge 100, 346 und 1513 Basenpaaren (bp) verwendet und nach der Reaktion mit Streptavidin-Cy5 angefärbt wurden. Die Anzahl an DNA Fragmente pro Well war auf berechnet. Der Graph zeigt die Anzahl an positiven Signalen gemessen an der Gesamtheit aller befüllten Kavitäten (10.000 Wells insgesamt, Fläche von 10 mm²) in Abhängigkeit der Länge der DNA Fragmente. Für das 100 bp DNA Fragment sind 95.3 ± 1.8 %, für das 346 bp DNA Fragment 92.1 ± 1.9 % und für das 1513 bp DNA Fragment 90.9 ± 3.5 % positive Signale gezählt. Somit ist gezeigt, dass man mit diesem System in der Lage ist auch DNA Fragmente >> 1000 bp zu amplifizieren und immobilisieren, was ein interessantes Feature im Vergleich zu den Limitierungen der emPCR darstellt.

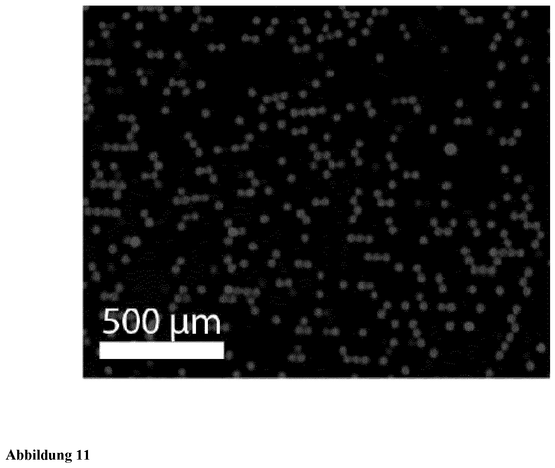

Abb. 11 zeigt beispielsweise einen mit Streptavidin-Cy5 eingefärbten Deckel nach der Reaktion in einem Multiwell-Array. Der Amplifikationsmix enthielt 0.025 DNA Fragmente pro Well. Der Deckel zeigt ein Muster an diskreten Signalen die von Wells stammen in denen ein DNA Fragment vorhanden war. Somit ist gezeigt dass das ein PCR-Produkt basierend auf einem einzigen DNA Fragment generiert, unter Beibehalt der räumlichen Verteilung an immobilisierte Fängermoleküle angebunden und visualisiert werden kann.

### Beispiel 1:

Wesentlicher Ablauf des erfindungsgemäßen Verfahrens

Im ersten Schritt wird ein Reaktionsmix angesetzt, der eine definierte Menge an Probenfragmenten enthält. Die Konzentration der Probenfragmente ist üblicher Weise so eingestellt, dass weniger als 1 Probenfragment pro Volumen einer Kavität vorliegt. Der Mix wird nun in ein Multiwell-Array eingebracht, dessen Oberfläche mit Fängermolekülen beschichtet ist. Durch Verschluss (Deckeln) der Mikrokavitäten werden diese gegeneinander isoliert, so dass in jeder Kavität eine unabhängige Amplifikationsreaktion stattfinden kann. Die Amplifikate werden üblicher Weise durch den einen Adaptor an die an der Oberfläche befindlichen Fängermoleküle gebunden. Somit wird die Innenseite jeder Kavität, in die ein Probenfragment eingebracht wurde, mit den entsprechenden Amplifikaten beschichtet. Als Resultat entsteht eine räumlich klar definierte und gegeneinander abgegrenzte Anordnung von Amplifikaten, welche der ursprünglichen Verteilung der einzelnen Probenfragmente entspricht.

### Beispiel 2:

Auf die Abdeckung, insbesondere die Hybriddeckel, die aus einer sehr dünnen Schicht (~ 5 µm) PDMS auf einen Glasträger bestehen können, werden nach folgender Prozedur die Fängermoleküle angebracht. Bevorzugt erfolgt zunächst eine Behandlung mit einem Sauerstoffplasma (40 kHz Generator, 100 Watt Leistung, 1 Minute Behandlungsdauer) um Hydroxidgruppen zu erzeugen. Im nächsten Schritt kann hieran das Aminosilan APTES gebunden (5% 3-aminopropyltriethoxysilane (APTES), 5 % DI Wasser, 90 % Ethanol, Reaktion übernacht bei Raumtemperatur) werden. An die nun an der Oberfläche befindlichen Aminogruppen wird bevorzugt der homobifunktionelle Linker 1,4-phenylene diisothiocyanate (PDITC) angebunden (0,15 g PDITC, 5 ml Pyridin, 45 ml Dimethylformamid (DMF)). Nachfolgend kann als Fängermolekül insbesondere eine modifizierte ssDNA-Sequenz angebunden (in 150 mM Natriumphosphatpuffer, pH = 8,3, Reaktionsdauer: übernacht bei Raumtemperatur) werden. Diese enthält am 5' Ende insbesondere einen Aminolinker, gefolgt von einer sechsteiligen Kohlenstoffkette (C6), gefolgt von 10 Thyminen. Jetzt folgt die eigentliche DNASequenz des DNA-Fängermoleküls. Der Amplifikationsmix enthält bevorzugt 30 U HotStar-Taq Plus, 1 × Reaktionspuffer, 1.5 mM MgCl2, jeweils 300 µM dATP, dGTP, dCTP, 225 µM dTTP, 75 µM biotin-dUTP, 0.5 % BSA, 0.05 % Tween 80, 0.125 µM Forwärtsprimer 5' - CTG AGC GGG CTG GCA - 3' und 1.000 µM Rückwärtsprimer 5' - GCC TCC CTC GCG CCA TCA G - 3', und zwischen 0 und 20 Kopie eines DNA-Fragments (pTYB1 Plasmid). Die genannten Komponenten werden mit hochreinen DNAse/RNAse-freiem, doppelt destillierten Wasser auf 16 µL aufgefüllt. Der Reaktionsmix wird auf Eis gelagert bis zum Befüllen des Multwell-Arrays. Eine Befüllvorrichtung um den Amplifikationsmix in das Array zu verbringen, ist teilidentisch mit der aus Abb. 7. Das Multiwell-Array ist vorzugsweise in eine PicoTiterPlate (Roche Diagnostics), aus der 13 × 13 mm² große Stücke herausgesägt werden (Abb. 7, A).

Das Array wird in die Vertiefung der Befüllvorrichtung eingelegt (Abb. 7B), worauf bevorzugt ein 500 µm dick strukturierte Gummilippe gelegt wird. Auf diese kann beispielsweise mit zwei M3 Schrauben ein mikrostrukturierter Befülldeckel fixiert, der je einen Ein- und Auslass besitzt. Nun können 16 µL Amplifikationsmix in die Kammer eingebracht werden. Die gesamte Befüllvorrichtung wird auf einen Ausschwingrotor einer Zentrifuge (Multifuge 3SR Plus, Heraeus, VWR, Bruchsal, Germany) eingelegt und bei 2200 rpm für 2 Minuten zentrifugiert.

Nach Zentrifugation wird das befüllte Array mit einem mit Fängermolekülen beschichtetem Deckel (Abb.7, C) abgedeckt. Hierauf wir nun ein Gummistempel gelegt (Abb. 7, D), darauf eine Klemmdeckel (Abb. 7, E) der nachfolgend mit zwei M3 Schrauben verschraubt wird. Die Reaktionsvorrichtung wird bevorzugt bei Raumtemperatur gelagert (1 ≤ t ≤ 10 Minuten) bevor dies in einen Slidecycler transferiert wird (peqStar in-situ, PEQLAB Biotechnologie). Nach der Amplifikationsreaktion, insbesondere der PCR, wird die Vorrichtung auseinandergebaut und alle Teile außer dem Deckel werden bevorzugt in DNA Exitus Plus (AppliChem) für mindestens 1 Stunde dekontaminiert. Das oberflächen-gebundene Produkt, bevorzugt das PCR-Produkt kann nun detektiert werden durch die Reaktion mit insbesondere streptavidin-Cy5 (5 µg·ml-1 streptavidin-Cy5 in 100 mM Natriumphosphate Puffer (dinatriumhydrogenphosphatdihydrat und natrium-dihydrogenphosphatemonohydrate, pH = 7.2, 0.1 % Tween 80, für 5 Minuten bei Raumtemperatur) oder einer Hybridisierungsreaktion (0.1 µM der Hybridisierungssonde in 5 × SSC, 50 % Formamid, 0.1 %, Reaktionsdauer: (12 h ≤ t ≤ 16 h) bei 42 °C).

Gewaschene Deckel können beispielsweise in dem Scanner InnoScan710 von Innopsys gescannt werden. Ergebnisse einer solchen Reaktion sind in Abb. 8 - 11 beispielhaft gezeigt. Hierbei wurde das PCR-Produkt auf die auf einem Deckel befindlichen Fängermoleküle angebunden und nach der jeweils aufgezeigten Methode visualisiert. Außer in Abb. 11 wurden bei allen Reaktionen 20 Moleküle pro Well in das Array eingebracht.

### Bezugszeichen

- 4: Probenfragment
- 5: Fängermolekül
- 6: hydrophobe Oberfläche
- 7: hydrophile Oberfläche
- 8: Oberfläche 1
- 9: Oberfläche 2
- 10: wässrige Phase
- 11: Öl-Phase
- 12: Deckel
- 13: Öl
- 14: Kraftfeld
- 15: Phasenübergang --> fest
- 19: Ventil schaltung
- 20: Fängermolekül
- 21: zusätzliche Nukleinsäuresequenz
- 22: Adaptor 1
- 23: Adaptor 2
- 24: Sequenz 1
- 25: Sequenz 2

## Patentansprüche

1. Verfahren zur Amplifikation von Nukleinsäuren, umfassend folgende Verfahrensschritte:
a. Bereitstellung einer Festphase mit Mikrostruktur, wobei die Mikrostruktur die Festphase so unterteilt, dass mehrere Kompartimente entstehen und wobei Fängermoleküle mit der Festphase verbunden sind und wobei es sich bei der Festphase um eine Pikotiterplatte handelt,
b. Einbringung mindestens eines Probenfragmentes in mindestens ein Kompartiment,
wobei das mindestens eine Probenfragment an beiden Enden Adaptoren aufweist, wobei die Adaptoren eine einheitliche Sequenz mit einer Länge von bis zu 1000 Nukleotiden und/oder unterschiedliche funktionelle Einheiten aufweisen,
c. Einbringung eines Reaktionsmixes für die Amplifikation in mindestens ein Kompartiment,
d. Abdeckung der einzelnen Kompartimente mit einer Abdeckung auf der Fängermoleküle immobilisiert sind,
e. Amplifikationsschritt, wobei jedes Probenfragment in dem entsprechenden abgeschlossenen Kompartiment eigenständig amplifiziert wird,
f. wobei die durch den Amplifikationsschritt generierten Amplifikate an den Fängermolekülen immobilisiert werden
wobei die Amplifikate nach der Immobilisierung an der Festphase sequenziert werden.

2. Verfahren nach Anspruch 1, wobei die Amplifikate analysiert und/oder derivatisiert werden.

3. Verfahren nach Anspruch 1 oder 2, wobei die Fängermoleküle Primer sind, bevorzugt Primer, die für den Amplifikationsschritt verwendet werden und/oder wobei die Amplifikate über kovalente Bindungen an die Fängermoleküle immobilisiert werden.

4. Verfahren nach einem oder mehreren der vorherigen Ansprüche, wobei die Kompartimente Kavitäten sind.

5. Verfahren nach einem oder mehreren der vorherigen Ansprüche, wobei die Einbringung des Probenfragments und die Einbringung des Reaktionsmixes in einem Schritt erfolgen, bevorzugt indem das Probenfragment vor der Einbringung mit dem Reaktionsmix vermischt wird.

6. Verfahren nach einem oder mehreren der vorherigen Ansprüche, wobei die Oberflächen der Festphase aus halbleitenden, amorphen, kristallinen und/oder faserartigen Materialien hergestellt sind und/oder,
wobei die Oberflächen hydrophile und/oder hydrophobe Bereiche aufweisen und/oder ein- oder mehrschichtig als strukturierte Fläche aufgebaut sind.

7. Verfahren nach einem oder mehreren der vorherigen Ansprüche, wobei die Abdeckung und/oder Abtrennung der Kompartimente Öl, elektrische Kräfte, Phasenübergänge, inerte Bereiche und/oder Deckel umfasst.

8. Verfahren nach einem oder mehreren der vorherigen Ansprüche, wobei die Fängermoleküle ein Spacermolekül aufweisen und/oder an einem oder mehreren Enden eine chemische Kopplungsgruppe enthalten und/oder
wobei die Fängermoleküle vorzugsweise eine Länge von bis zu 1000 Nukleotiden aufweisen.

9. Verfahren nach einem oder mehreren der vorherigen Ansprüche, wobei die Fängermoleküle mindestens eine Anbindestelle für Probenfragmente und/oder Amplifikate umfassen und/oder
wobei die Fängermoleküle und/oder die Amplifikate eine photoaktivierbare Spaltgruppe aufweisen.

10. Verfahren nach einem oder mehreren der vorherigen Ansprüche,
wobei die Fängermoleküle eine Promotorbindestelle für eine RNA-Polymerase oder ein zellfreies Expressionssystem enthalten.

11. Verfahren nach einem oder mehreren der vorherigen Ansprüche, wobei die Probenfragmente DNA oder RNA, sind und/oder wobei die Probenfragmente direkt oder indirekt über ein eingebautes Label aus DNA oder RNA amplifiziert werden.

12. Verfahren nach einem oder mehreren der vorherigen Ansprüche, wobei die Probenfragmente in einer Länge von bis zu 10¹⁰ Nukleotiden einzelsträngig oder doppelsträngig vorliegen.

13. Verfahren nach einem oder mehreren der vorherigen Ansprüche, wobei der Amplifikationsschritt insbesondere aus der Gruppe umfassend PCR, RPA, RCA, SDA, NASBA oder LAMP ausgewählt ist und/oder
wobei der Amplifikationsschritt keine Öl-Emulsions-PCR ist.

14. Vorrichtung für die Durchführung eines Verfahrens nach einem oder mehreren der vorherigen Ansprüche, umfassend
g) eine Festphase in Form eine Pikotiterplatte mit mehreren Kompartimenten, wobei Fängermoleküle an die Oberfläche der Festphase, bevorzugt der Kompartimente gebunden sind und
h) eine Abdeckung,
wobei Fängermoleküle an die Abdeckung gebunden sind.

15. Kit zur Amplifikation von Nukleinsäuren und/oder Herstellung eines Arrays umfassend
k) eine Festphase in Form einer Pikotiterplatte mit mehreren Kompartimenten, wobei Fängermoleküle an die Oberfläche der Festphase, bevorzugt der Kompartimente gebunden sind,
l) eine Abdeckung und
m) mindestens einen Reaktionsmix,
wobei Fängermoleküle an die Abdeckung gebunden sind.

## Claims

1. Method for the amplification of nucleic acids, comprising the following method steps:
a. providing a solid phase having a microstructure, wherein the microstructure divides the solid phase so that multiple compartments result and wherein capture molecules are connected to the solid phase, and wherein the solid phase is a picotiter plate,
b. introducing at least one sample fragment into at least one compartment,
wherein the at least one sample fragment has adapters at both ends, wherein the adapters have a uniform sequence having a length of up to 1000 nucleotides and/or have different functional units,
c. introducing a reaction mix for the amplification into at least one compartment,
d. covering the individual compartments using a cover on which the capture molecules are immobilized,
e. amplification step, whereby each sample fragment is independently amplified in the corresponding closed compartment,
f. wherein the amplicons generated by the amplification step are immobilized on the capture molecules
wherein the amplicons are sequenced after immobilization on the solid phase.

2. Method according to Claim 1, wherein the amplicons are analysed and/or derivatised.

3. Method according to Claim 1 or 2, wherein the capture molecules are primers, preferably primers, which are used for the amplification step and/or
wherein the amplicons are immobilized on the capture molecules via covalent bonds.

4. Method according to any one or more of the preceding claims, wherein the compartments are cavities.

5. Method according to any one or more of the preceding claims, wherein the introduction of the sample fragment and the introduction of the reaction mix take place in one step, preferably by the sample fragment being mixed with the reaction mix before the introduction.

6. Method according to any one or more of the preceding claims, wherein the surfaces of the solid phase are produced from semiconducting, amorphous, crystalline, and/or fibrous materials and/or,
wherein the surfaces have hydrophilic and/or hydrophobic regions and/or are built up in one or more layers as a structured surface.

7. Method according to any one or more of the preceding claims, wherein the cover and/or partition of the compartments comprises oil, electrical forces, phase transitions, inert regions, and/or covers.

8. Method according to any one or more of the preceding claims, wherein the capture molecules have a spacer molecule and/or contain a chemical coupling group at one or more ends and/or wherein the capture molecules preferably have a length of up to 1000 nucleotides.

9. Method according to any one or more of the preceding claims, wherein the capture molecules comprise at least one attachment point for sample fragments and/or amplicons and/or
wherein the capture molecules and/or the amplicons have a photoactivatable cleavage group.

10. Method according to any one or more of the preceding claims,
wherein the capture molecules contain a promoter binding site for an RNA polymerase or a cell-free expression system.

11. Method according to any one or more of the preceding claims, wherein the sample fragments are DNA or RNA and/or wherein the sample fragments are amplified directly or indirectly via a built-in label made of DNA or RNA.

12. Method according to any one or more of the preceding claims, wherein the sample fragments are present in a length of up to 10¹⁰ nucleotides in single-stranded or double-stranded form.

13. Method according to any one or more of the preceding claims, wherein the amplification step is selected in particular from the group comprising PCR, RPA, RCA, SDA, NASBA, or LAMP and/or wherein the amplification step is not an oil emulsion PCR.

14. Device for carrying out a method according to any one or more of the preceding claims, comprising
g) a solid phase in the form of a picotiter plate having multiple compartments, wherein capture molecules are bound to the surface of the solid phase, preferably the compartments, and
h) a cover,
wherein capture molecules are bound to the cover.

15. Kit for the amplification of nucleic acids and/or production of an array comprising
k) a solid phase in the form of a picotiter plate having multiple compartments, wherein capture molecules are bound to the surface of the solid phase, preferably the compartments,
1) a cover, and
m) at least one reaction mix,
wherein capture molecules are bound to the cover.

## Revendications

1. Procédé d'amplification d'acides nucléiques, comprenant les étapes de procédé suivantes :
a. la fourniture d'une phase solide avec une microstructure, dans lequel la microstructure divise la phase solide de sorte que plusieurs compartiments apparaissent, et dans lequel des molécules de capture sont attachées à la phase solide, et dans lequel la phase solide est une plaque de picotitre,
b. l'introduction d'au moins un fragment d'échantillon dans au moins un compartiment,
dans lequel l'au moins un fragment d'échantillon présente des adaptateurs aux deux extrémités, dans lequel les adaptateurs présentent une séquence uniforme avec une longueur allant jusqu'à 1000 nucléotides et/ou des unités fonctionnelles différentes,
c. l'introduction d'un mélange réactionnel pour l'amplification dans au moins un compartiment,
d. le recouvrement des compartiments individuels avec une couverture sur laquelle sont immobilisées les molécules de capture,
e. l'étape d'amplification, dans lequel chaque fragment d'échantillon est indépendamment amplifié dans le compartiment fermé correspondant,
f. dans lequel les amplicons générés par l'étape d'amplification sont immobilisés sur les molécules de capture dans lequel les amplicons sont séquencés après l'immobilisation sur la phase solide.

2. Procédé selon la revendication 1, dans lequel les amplicons sont analysés et/ou sont dérivés.

3. Procédé selon la revendication 1 ou 2, dans lequel les molécules de capture sont des amorces, de préférence des amorces utilisées pour l'étape d'amplification, et/ou
dans lequel les amplicons sont immobilisés sur les molécules de capture par l'intermédiaire de liaisons covalentes.

4. Procédé selon l'une ou plusieurs des revendications précédentes, dans lequel les compartiments sont des cavités.

5. Procédé selon l'une ou plusieurs des revendications précédentes, dans lequel l'introduction du fragment d'échantillon et l'introduction du mélange réactionnel ont lieu en une seule étape, de préférence par le mélange du fragment d'échantillon avec le mélange réactionnel avant l'introduction.

6. Procédé selon l'une ou plusieurs des revendications précédentes, dans lequel les surfaces de la phase solide sont constituées de matériaux semi-conducteur, amorphes, cristallins et/ou fibreux, et/ou
dans lequel les surfaces présentent des zones hydrophiles et/ou hydrophobes et/ou sont construites en une ou en plusieurs couches comme une surface structurée.

7. Procédé selon l'une ou plusieurs des revendications précédentes, dans lequel la couverture et/ou la séparation des compartiments comprend de l'huile, des forces électriques, des transitions de phase, des zones inertes et/ou une couverture.

8. Procédé selon l'une ou plusieurs des revendications précédentes, dans lequel les molécules de capture présentent une molécule espaceur et/ou contiennent un groupe de couplage chimique à une ou à plusieurs extrémités et/ou
dans lequel les molécules de capture présentent de préférence une longueur allant jusqu'à 1000 nucléotides.

9. Procédé selon l'une ou plusieurs des revendications précédentes, dans lequel les molécules de capture comprennent au moins un point de fixation pour des fragments d'échantillon et/ou des amplicons
dans lequel les molécules de capture et/ou les amplicons présentent un groupe de clivage photoactivable.

10. Procédé selon l'une ou plusieurs des revendications précédentes,
dans lequel les molécules de capture contiennent un site de liaison promoteur pour une ARN polymérase ou un système d'expression acellulaire.

11. Procédé selon l'une ou plusieurs des revendications précédentes, dans lequel les fragments d'échantillon sont de l'ADN ou de l'ARN et/ou dans lequel les fragments d'échantillon sont amplifiés directement ou indirectement via un marqueur intégré à partir d'ADN ou d'ARN.

12. Procédé selon l'une ou plusieurs des revendications précédentes, dans lequel les fragments d'échantillon sont présents sous forme simple brin ou double brin dans une longueur allant jusqu'à 10¹⁰ nucléotides.

13. Procédé selon l'une ou plusieurs des revendications précédentes, dans lequel l'étape d'amplification est choisie en particulier dans le groupe comprenant PCR, RPA, RCA, SDA, NASBA ou LAMP et/ou
dans lequel l'étape d'amplification n'est pas une PCR en émulsion huileuse.

14. Dispositif pour la mise en oeuvre d'un procédé selon l'une ou plusieurs des revendications précédentes, comprenant
g) une phase solide sous la forme d'une plaque de picotitre à plusieurs compartiments, dans lequel les molécules de capture sont fixées à la surface de la phase solide, de préférence des compartiments, et
h) une couverture,
dans lequel les molécules de capture sont liées à la couverture.

15. Kit d'amplification d'acides nucléiques et/ou fabrication d'une matrice comprenant
k) une phase solide sous la forme d'une plaque de picotitre à plusieurs compartiments, dans lequel les molécules de capture sont fixées à la surface de la phase solide, de préférence des compartiments,
l) une couverture et
m) au moins un mélange réactionnel,
dans lequel les molécules de capture sont liées à la couverture.
